(19) Europäisches Patentamt
European Patent Office
Office européen des brevets

(11) Veröffentlichungsnummer: **0 126 711**
**B1**

(12) # EUROPÄISCHE PATENTSCHRIFT

(45) Veröffentlichungstag der Patentschrift: **02.08.89**

(21) Anmeldenummer: **84810227.3**

(22) Anmeldetag: **10.05.84**

(51) Int. Cl.⁴: **C 07 D 239/47**, C 07 D 239/48, C 07 D 239/42, C 07 D 239/52, C 07 D 239/56, C 07 D 239/70, C 07 D 405/12, C 07 D 409/12, C 07 D 401/12, A 01 N 47/36

(54) **Herbizid wirksame und pflanzenwuchsregulierende Pyrimidin-Derivate, deren Herstellung und Verwendung.**

(30) Priorität: **16.05.83 CH 2636/83**

(43) Veröffentlichungstag der Anmeldung: **28.11.84 Patentblatt 84/48**

(45) Bekanntmachung des Hinweises auf die Patenterteilung: **02.08.89 Patentblatt 89/31**

(84) Benannte Vertragsstaaten: **BE CH DE FR GB IT LI NL**

(56) Entgegenhaltungen:
**EP-A-0 001 485**
**EP-A-0 023 141**
**EP-A-0 030 433**
**EP-A-0 044 212**
**EP-A-0 071 958**
**EP-A-0 125 864**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Meyer, Willy, Talweg 49, CH- 4125 Riehen (CH)**
Erfinder: **Hoegerle, Karl, Dr., Wasgenring 92, CH- 4055 Basel (CH)**
Erfinder: **Thummel, Rudolph C., Route d'Alle 424, CH- 2892 Courgenay (CH)**
Erfinder: **Tobler, Hans, Dr., Baselmattweg 157, CH- 4123 Allschwil (CH)**
Erfinder: **Böhner, Beat, Dr., Hügelweg 3, CH- 4102 Binningen (CH)**

EP 0 126 711 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft neue, herbizid wirksame und pflanzenwuchsregulierendeN-Pyrimidin-4-yl-N'-sulfonylharnstoffe, Verfahren zu deren Herstellung, sie als Wirkstoffe enthaltende Mittel, sowie deren Verwendung zum Bekämpfen von Unkräutern, vor allem in Nutzpflanzenkulturen oder zum Regulieren und Hemmen des Pflanzenwachstums.

Die erfindungsgemässen N-Pyrimidin-4-yl-N'-sulfonylharnstoffe entsprechen der Formel I

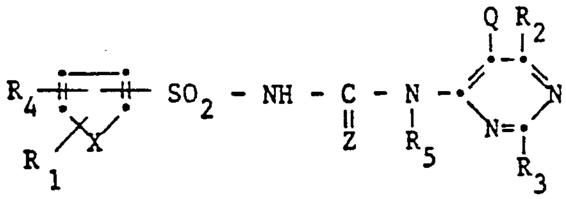

(I),

worin

Q Halogen, $C_1$-$C_4$ Alkyl, $C_1$-$C_4$ Halogenalkyl, $C_1$-$C_4$ Alkoxy, $C_1$-$C_4$-Halogenalkoxy, Amino, $C_1$-$C_4$ Alkylamino, $C_1$-$C_4$ Dialkylamino, $C_4$ Alkoxycarbonyl, Formyl, $C_2$-$C_4$ Alkoxyalkyl, $C_1$-$C_4$-Alkylthio, $C_1$-$C_4$ Alkylsulfinyl, $C_4$ Alkylsulfonyl, $C_1$-$C_4$-Alkenyl oder $C_1$-$C_4$-Alkinyl

$R_1$ Wasserstoff, Halogen, Nitro oder einen Rest

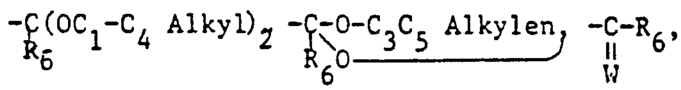

$-SO_2NR_7R_8$ oder $-COR_9$-oder $-(Y)_m-R_{10}$,

$R_2$ und $R_3$ unabhängig voneinander Wasserstoff, Halogen, $C_1$-$C_4$ Alkyl, $C_1$-$C_4$ Alkoxy, $C_1$-$C_4$ Halogenalkyl, $C_1$-$C_4$ Halogenalkoxy, $C_1$-$C_4$ Alkylthio, $C_2$-$C_4$ Alkoxyalkyl, $C_2$-$C_4$ Alkoxyalkoxy, Amino, $C_1$-$C_4$ Alkylamino, $C_1$-$C_4$ Dialkylamino oder Cyclopropyl, wobei Q und $R_2$ zusammen auch eine 2 - 4 gliedrige Kohlenstoff-Kette bilden können, welche gegebenenfalls noch ein Sauerstoff- oder Schwefelatom oder die Gruppe N-$R_5$ enthalten kann,

$R_4$ Wasserstoff, Halogen, $C_1$-$C_4$ Alkyl, Methoxy, Nitro oder Trifluormethyl,

$R_5$ Wasserstoff oder $C_1$-$C_4$ Alkyl oder $C_3$-$C_4$ Alkenyl,

X Sauerstoff, Schwefel, $-NR_5-$, 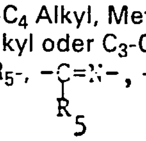, $-CH=CH-$ oder einen annelierten Phenylring,

Z Sauerstoff oder Schwefel,

$R_6$ Wasserstoff, $C_1$-$C_4$ Alkyl, $C_1$-$C_4$-Halogenalkyl, $C_3$-$C_6$ Cycloalkyl, $C_4$-$C_7$ Cycloalkylalkyl oder $C_2$-$C_4$ Alkoxyalkyl,

$R_7$ Wasserstoff, $C_1$-$C_4$ Alkyl, $C_1$-$C_4$-Cyanoalkyl, Methoxy oder Äthoxy,

$R_8$ dasselbe wie $R_5$ oder

$R_7$ und $R_8$ zusammen mit dem sie bindenden Stickstoffatom einen 5 - 6 gliedrigen gesättigten, gegebenenfalls ein Sauerstoff- oder Schwefelatom als Ringglied enthaltenden, Heterocyclus,

$R_9$ $C_1$-$C_4$ Alkoxy, $C_3$-$C_6$ Alkenyloxy, $C_3$-$C_6$ Alkinyloxy, $C_2$-$C_6$-Halogenalkoxy, $C_1$-$C_4$ Cyanoalkoxy, $C_1$-$C_4$ Alkylthio, $C_3$-$C_4$ Alkenylthio, $C_3$-$C_4$ Alkinylthio, $C_5$-$C_6$ Cycloalkoxy, $C_4$-$C_7$ Cycloalkylalkoxy, $-NR_7R_8$ oder $C_2$-$C_6$ Alkoxyalkoxy,

$R_{10}$ $-C_1$-$C_4$ alkyl, unsubstituiert oder cin- oder mehrfach durch Halogen, Cyano, Methoxy, Äthoxy, Nitro, $C_1$-$C_2$Alkyl $S(O)_n$, $C_1$-$C_2$ Halogenalkoxy, $C_1$-$C_2$ Halogenalkylthio, $-COR_6$, $-COR_9$, $-SO_2NR_7R_8$ $-C_2$-$C_4$ Alkenyl unsubstituiert oder ein- oder mehrfach substituiert durch Halogen, Nitro, Cyan, Methoxy, Äthoxy oder $C_1$-$C_2$ Alkyl$S(O)_n$, $-C_2$-$C_4$ Alkinyl,

m 0 oder 1,

n 0, 1 oder 2

W Sauerstoff oder die Gruppe $=N-O-R_5$ und

Y Sauerstoff, Schwefel -SO- oder $-SO_2-$

bedeuten, mit der Massgabe, dass im Rest $R_1$ in der Gruppe $-(Y)_m-R_{10}$, $R_{10}$ bloss $C_3$-$C_4$ Alkinyl ist, wenn m die Zahl 1 bedeutet.

Die Erfindung umfasst auch die Salze dieser Sulfonylharnstoffe.

In der 5-Stellung des Pyrimidinrings unsubstituierte N-Pyrimidin-4-yl-N'-sulfonylharnstoffe mit herbizider Wirkung sind bekannt, z. B. aus den europäischen Offenlegungsschriften EP-A-1 485, EP-A-13 480 EP-A-44 212 EP-A-23 141 EP-A-30 433 und EP-A-71 958.

In der 5-Stellung substituierte N-Pyrimidin-2-yl-N'-sulfonylharnstoffe mit herbizider Wirkung sind aus der US-PS-4 342 587 bekannt.

Ferner werden in der EP-A-61 661 in der 5-Stellung substituierte N-Pyrimidin-4-yl-N-Alkylsulfonylharnstoffe mit Herbizidwirkung beschrieben.

Des weiteren sind aus der nicht vorveröffentlichten europäischen Patentanmeldung EP-125 864 N-Pyrimidin-4-ylsulfonylharnstoffe bekannt geworden, welche in der 5-Position des Pyrimidinsystems durch CN, $C_1$-$C_3$-Alkoxycarbonyl, $NO_2$, $C_1$-$C_3$-Alkylsulfinyl oder $C_1$-$C_3$-Alkylsulfonyl substituiert sind.

Die vorliegenden N-Pyrimidin-4-yl-sulfonylharnstoffe, welche in der 5-Stellung des Pyrimidinrings durch einen Rest Q substituiert sind, sind neu. Sie zeichnen sich durch ausgezeichnete Herbizid-, Selektivherbizid- und den Pflanzenwuchs regulierende Wirkung aus, wobei die gewünschte Wirkung bereits mit Aufwandmengen von weniger als 1 kg pro Hektar erreicht wird.

In den Definitionen von $R_1$ bis $R_{11}$ ist ein Alkylrest geradkettig oder verzweigt; z. B. Methyl, Äthyl, n-Propyl, i-Propyl, die vier isomeren Butyl, n-Amyl, i-Amyl, 2-Amyl oder 3-Amyl, vorzugsweise ist der Alkylrest jedoch geradkettig.

Unter Alkoxy in den Definitionen ist zu verstehen: Methoxy, Äthoxy, n-Propyloxy, i-Propyloxy und die vier isomeren Butyloxyreste, insbesondere aber Methoxy oder Äthoxy.

Beispiele für Alkylthio sind Methylthio, Äthylthio, n-Propylthio, i-Propylthio und n-Butylthio, insbesondere aber Methylthio und Äthylthio.

Beispiele für Alkenylreste sind Vinyl, Allyl, Isopropenyl, 1-Propenyl, 1-Butenyl, 2-Butenyl, 3-Butenyl, 1-Isobutenyl, 2-Isobutenyl, 1-Pentenyl, 2-Pentenyl, 3-Pentenyl und 4-Pentenyl, insbesondere aber Vinyl, Allyl und 4-Pentenyl.

Alkinylreste sind beispielsweise Äthinyl, Propargyl, 1-Propinyl, die drei isomeren Butinylreste sowie 4-Pentinyl, vorzugsweise jedoch der Propargylrest.

Unter Halogen ist in den obigen Definitionen sowie in Halogenalkyl, Fluor, Chlor, Brom und Jod, vorzugsweise jedoch Fluor und vor allem Chlor zu verstehen.

In den Definitionen bedeutet Cycloalkyl z. B. Cyclopropyl, Cyclobutyl, Cyclopentyl oder Cyclohexyl, vorzugsweise aber Cyclopropyl, Cyclopentyl oder Cyclohexyl.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Äthylamin, Propylamin, i-Propylamin, die vier isomeren Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin, und i-Chinolin, insbesondere aber Äthyl-, Propyl-, Diäthyl- oder Triäthylamin, vor allem aber Isopropylamin und Diäthanolamin.

Beispiele für quaternäre Ammoniumbasen sind im allgemeinen die Kationen von Halogenammoniumsalzen, z. B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Die besten Wirkungen wurden mit den Verbindungen erzielt, welche der folgenden Formel entspricht:

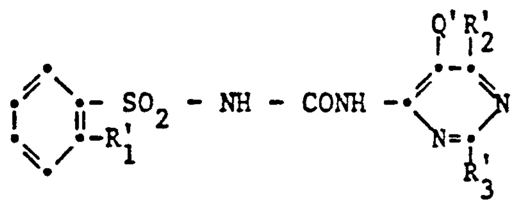

(Ia) ,

worin Q', $R_1$' und $R_2$' die oben für Q, $R_1$ und $R_2$ gegebene Bedeutung haben, während $R_3$' $C_1$-$C_2$ Alkyl, $C_1$-$C_2$ Halogenalkyl, Cyclopropyl, $C_1$-$C_3$ Alkoxy, $C_1$-$C_2$ Halogenalkoxy, Methylthio oder Difluormethylthio bedeutet.

Speziell bewährt haben sich die Verbindungen der Formel Ia, in denen

Q' Halogen, $C_1$-$C_4$ Alkyl, Amino, Formyl, Methylthio oder Methylsulfinyl,

$R_1$' Halogen, Nitro, $C_1$-$C_4$-Halogenalkyl, $C_1$-$C_4$-Alkoxy, $C_1$-$C_4$-Halogenalkoxy, $C_3$-$C_4$-Alkenyloxy, $C_2$-$C_4$-Halogenalkenyloxy, $C_1$-$C_4$-Halogenalkylthio, $C_1$-$C_4$-Alkoxycarbonyl, $C_1$-$C_4$-Alkylsulfonyl oder Dimethylsulfamoyl

$R_2$' Halogen, $C_1$-$C_3$, Alkyl, Cyclopropyl, $C_1$-$C_3$ Alkoxy, $C_1$-$C_2$ Halogenalkoxy, Amino, Methylamino, Dimethylamino oder Difluormethylthio,

$R_3$' $C_1$-$C_2$ Alkyl, $C_1$-$C_2$ Halogenalkyl, Cyclopropyl, $C_1$-$C_3$ Alkoxy, $C_1$-$C_2$-Halogenalkoxy, Methylthio oder Difluormethylthio bedeuten, insbesondere aber diejenigen, in denen Q' die obige Bedeutung hat, während

$R_1$' Halogen, Nitro, $C_1$-$C_4$ Halogenalkyl, $C_1$-$c_4$ Alkoxy, $C_1$-$C_4$ Halogenalkoxy, $C_1$-$C_4$ Halogenalkylthio, $C_1$-$C_4$ Alkoxycarbonyl oder Dimethylsulfamoyl,

$R_2$' Halogen, $C_1$-$C_2$ Alkyl, $C_1$-$C_2$ Alkoxy, $C_1$-$C_2$ Halogenalkoxy, Methylamino oder Dimethylamino, und

$R_3$' $C_1$-$C_2$ Alkoxy, Trifluoräthoxy oder Difluormethoxy bedeuten.

Folgende Einzelverbindungen sind durch ihre gute Wirkung aufgefallen:

N-(2-Methoxycarbonylphenylsulfonyl)-N'-(2,6-dimethoxy-5-methyl-pyrimidin-4-yl)-harnstoff,
N-(2-Difluormethoxyphenylsulfonyl)-N'-(2,6-dimethoxy-5-methylpyrimidin-4-yl)-harnstoff.

Die Herstellung der Verbindungen der Formel I erfolgt in an sich bekannter Weise in einem inerten, organischen Lösungsmittel.

Nach einem ersten Verfahren werden die Verbindungen der Formel erhalten, indem man ein Sulfonamid der Formel II

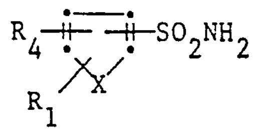

(II) ,

worin $R_1$, $R_4$ und X die oben gegebene Bedeutung haben, in Gegenwart einer Base mit einem Pyrimidin-4-yl-carbamat der Formel III umsetzt

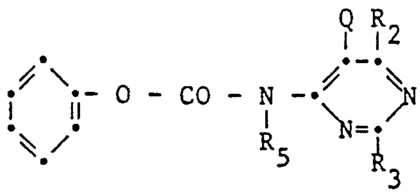

(III) ,

worin Q, $R_2$, $R_3$ und $R_5$ die unter Formel I gegebene Bedeutung haben, umsetzt und gegebenenfalls in ein Salz überführt.

Nach einem zweiten Verfahren gelangt man zu Verbindungen der Formel I, indem man ein Phenylsulfonylisocyanat der Formel IV

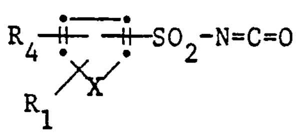

(IV),

worin $R_1$, $R_4$ und X die oben gegebene Bedeutung haben, gegebenenfalls in Gegenwart einer Base mit einem Amin der Formel V umsetzt

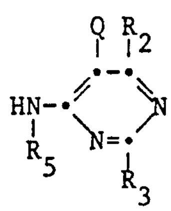

(V),

worin Q, $R_2$, $R_3$ und $R_5$ die oben gegebene Bedeutung haben, und gegebenenfalls in ein Salz überführt.

Nach einem weiteren Verfahren werden die Verbindungen der Formel I hergestellt, in denen $R_5$ Wasserstoff bedeutet, indem man ein Sulfonamid der oben angegebenen Formel II gegebenenfalls in Gegenwart einer Base mit einem Isocyanat der Formel VI umsetzt

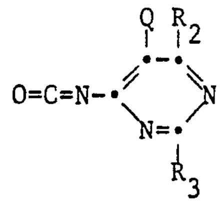

(VI) ,

worin Q, $R_2$ und $R_3$ die oben gegebene Bedeutung haben, und gegebenenfalls in ein Salz überführt.

Schliesslich kann man die Verbindungen der Formel I auch erhalten, indem man ein N-Phenylsulfonylcarbamat der Formel VII

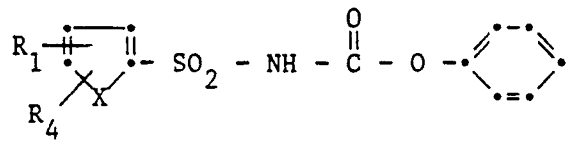

(VII) ,

worin $R_1$, $R_4$ und X die oben gegebene Bedeutung haben, mit einem Amin der oben gegebenen Formel V umsetzt und gegebenenfalls in ein Salz überführt.

Die erhaltenen Harnstoffe der Formel I können gegebenenfalls mittels Aminen, Alkalimetall- oder Erdalkalimetallhydroxiden oder quaternären Ammoniumbasen in basische Additionssalze übergeführt werden. Dies geschieht beispielsweise durch Umsetzen mit der äquimolaren Menge Base und Verdampfen des Lösungsmittels. Solche Umsetzungen sind bekannt und beispielsweise in den US Patentschriften Nr. 2 384 757 und 3 410 887 beschrieben.

Die Ausgangsstoffe der Formeln II bis VII sind zum Teil bekannt oder können nach allgemein bekannten Methoden hergestellt werden.

So können die Sulfonamide der Formel II aus den entsprechenden Aminen durch Diazotierung und Austausch der Diazogruppe mit Schwefeldioxid in Gegenwart eines Katalysators wie Kupfer-II-chlorid in Essigsäure, wobei das Sulfonylchlorid entsteht, siehe J.Org.Chem. 25, 1824 (1960), und nachfolgendes Umsetzen des entstandenen Sulfochlorides mit Ammoniumhydroxid-Lösung, gewonnen werden.

Die Sulfonylisocyanate der Formel IV können durch Umsetzungen der Sulfonamide der Formel II mit Phosgen in Anwesenheit von Butylisocyanat in einem chlorierten Kohlenwasserstoff als Lösungsmittel, bei Rückflusstemperatur erhalten werden. Siehe dazu "Newer Methods of Preparative Organic Chemistry" Band VI, 223-241, Academic Press New York und London.

Amine der Formel V sind bekannt und zum Teil im Handel erhältlich oder sie können nach bekannten Methoden hergestellt werden, siehe "The Chemistry of Heterocyclic Compounds" Band XVI + Suppl I, Interscience Publishers New York und London.

Durch Umsetzung von Aminen der Formel V mit Phosgen oder Oxalylchlorid in chlorierten Kohlenwasserstoffen als Lösungsmittel lassen sich die Isocyanate der Formel VI herstellen.

Die Sulfonylcarbamate der Formel VII werden durch Umsetzung der Sulfonamide der Formel II mit Diphenylcarbamat in Gegenwart einer Base erhalten, siehe Japanische Patentschrift 61 169.

In analoger Weise werden die Carbamate der Formel III aus den Aminen der Formel V gewonnen.

Diese Umsetzungen werden vorteilhafterweise in aprotischen, inerten, organischen Lösungsmitteln vorgenommen wie Methylenchlorid, Tetrahydrofuran, Acetonitril, Dioxan, Toluol.

Die Reaktionstemperaturen liegen vorzugsweise zwischen -20° und +120°C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können gegebenenfalls durch Zugabe einiger Tropfen Base oder Isocyanat als Reaktionskatalysatoren beschleunigt werden.

Die Endprodukte können durch Einengen und/oder Verdampfen des Lösungsmittels isoliert und durch Umkristallisation oder Zerreiben des festen Rückstandes in Lösungsmitteln, in denen sie sich nicht gut lösen, wie Äther, aromatischen Kohlenwasserstoffen oder chlorierten Kohlenwasserstoffen, gereinigt werden.

Die Wirkstoffe der Formel I sind stabile Verbindungen. Ihre Handhabung bedarf keiner vorsorglicher Massnahmen.

Die Verbindungen der Formel I zeichnen sich durch gute selektivherbizide Eigenschaften aus, die sie ausgezeichnet zum Einsatz in Kulturen von Nutupflanzen wie beispielsweise Weizen, Mais und Soja befähigen. Es werden dabei teilweise auch Unkräuter geschädigt, welchen bisher nur mit Totalherbiziden beizukommen war.

Die Wirkungsart dieser Herbizide ist unüblich, sie sind translozierbar, d. h. sie werden von den Pflanzen aufgenommen und an andere Stellen transportiert, wo sie dann zur Wirkung kommen. Das unübliche daran ist, dass sie nicht nur den Weg der Nährstoffe durch die Gefässbündel im Holzteil, von den Wurzeln in die Blätter nehmen, sondern auch durch die Siebröhren im Bastteil von den Blättern zurück in die Wurzel transloziert werden können. So gelingt es beispielsweise durch Oberflächenbehandlung perenierende Unkräuter bis in die

Wurzeln zu vernichten. Die neuen Verbindungen der Formel I wirken bereits bei - im Vergleich zu anderen Herbiziden - sehr geringen Aufwandmengen.

Die Verbindungen der Formel I haben ausserdem starke pflanzenwuchsregulierende Eigenschaften, die sich in einer Ertragssteigerung von Kulturpflanzen oder Erntegut auswirken kann. Viele Verbindungen der Formel I zeigen darüberhinaus eine konzentrationsabhängige pflanzenwuchshemmende Wirkung. Es werden sowohl Monokotyledonen als auch Dikotyledonen in ihrem Wachstum beeinträchtigt.

So können z. B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Bei monokotylen Pflanzen, z. B. Gräsern oder auch Kulturpflanzen wie Getreide, ist eine Hemmung des vegetativen Wachstums manchmal gewünscht und vorteilhaft. Eine derartige Wuchshemmung ist unter anderem bei Gräsern von wirtschaftlichem Interesse, denn dadurch kann z. B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Überlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens ("Lagerns") der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Weiter eignen sich die Verbindungen der Formel I, um das Keimen von eingelagerten Kartoffeln zu verhindern. Bei Kartoffeln entwickeln sich bei der Einlagerung über den Winter häufig Keime, die Schrumpfen, Gewichtsverlust und Faulen zur Folge haben.

Bei grösseren Aufwandmengen werden alle getesteten Pflanzen in ihrer Entwicklung so geschädigt, dass sie absterben.

Die Erfindung betrifft auch herbizide und pflanzenwuchsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmittel eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/ oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z. B. die Na- oder K-Salze der Öl- oder Stearinsäure, oder von natürlichen

Fettsäuregemischen, die z. B. aus Kokosnuss- oder Talgöl gewonnen werden können, genannt. Ferner sind auch die Fettsäure-methyl-taurinsalze zu erwähnen.

Häufig werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Äthylenoxid-Adduktes und Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi(2-chloräthyl)-äthylammonium-bromid.

Die in der Formulierungstechnik gebräuchlichen Tenside sind u. a. in folgenden Publikationen beschrieben:.

"Mc Cutcheon's Detergents and Emulsifiers Annual" MC
Publishing Corp., Ridgewood, New Jersey, 1979.
J. and M. Ash, "Encyclopedia of Surfactants" Vol I-III.
Chemical Publishing Co., Inc. New York, 1980/81
H. Stache "Tensid Taschenbuch" 2. Auflage C.Hanser Verlag, München und Wien, 1981.

Die pestiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

**Emulgierbare Konzentrate**

| | |
|---|---|
| Aktiver Wirkstoff: | 1 bis 20 %, bevorzugt 5 bis 10 % |
| oberflächenaktive Mittel: | 5 bis 30 %, bevorzugt 10 bis 20 % |
| flüssiges Trägermittel: | 50 bis 94 %, bevorzugt 70 bis 85 %. |

**Stäube**

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 %. |

**Suspensions-Konzentrat**

| | |
|---|---|
| Aktiver Wirkstoff: | 5 bis 75 %, vorzugsweise 10 bis 50 % |
| Wasser: | 94 bis 25 %, vorzugsweise 90 bis 30 % |

oberflächenaktives Mittel: 1 bis 40 %, vorzugsweise 2 bis 30 %.

**Benetzbare Pulver**

| | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 95 %, vorzugsweise 15 bus 90 %. |

**Granulate**

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 %. |

Während als Handelswerte eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,001 bis 10 kg/ha, vorzugsweise 0,025 bis 5 kg/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den nachfolgenden Beispielen sind Temperaturen in Celsiusgraden angegeben. Teile und Prozentangaben beziehen sich auf das Gewicht. Druckangaben beziehen sich auf Millibar (mb) (1 mb = 100 Pascal).

**Beispiel 1:**

Herstellung von N-(2-Methoxycarbonylphenylsulfonyl)-N,-(2,6-dimethoxy-5-methyl-pyrimidin-4-yl)-harnstoff

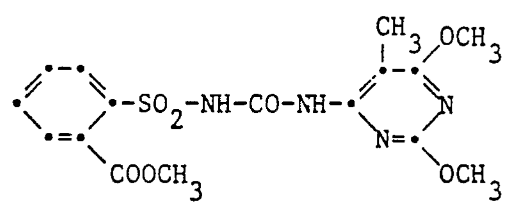

2,41 g 2-Methoxycarbonylphenylsulfonylisocyanat und 1,7 g 4-Amino-2,6-dimethoxy-5-methyl-pyrimidin werden mit 40 ml absolutem Dioxan während 2 Stunden bei 80 - 85° verrührt. Die gebildete Suspension wird vollständig eingedampft. Aus dem Rückstand erhält man durch Umkristallisieren aus einem Gemisch von Aceton/Äther 3,4 g N-(2-Carboxymethylphenyl-sulfonyl)-N'-(2,6-dimethoxy-5-methyl-pyrimidin-4-yl)-harnstoff vom Schmelzpunkt 195 - 196°.

Das als Zwischenprodukt benötigte 4-Amino-2,6-dimethoxy-5-methylpyrimidin wird wie folgt erhalten.:

a) Herstellung von 5-Methylbarbitursäure

In einem 5-Liter-Kolben werden 46 g (2 Mol) Natrium in 1 l absolutem Äthanol gelöst. Zu dieser Lösung gibt man zuerst 348 g (2 Mol) Methylmalonsäuremethylester und danach tropft man bei 70° unter Rühren eine Lösung von 120 g (2 Mol) Harnstoff in 1 l absolutem Äthanol innerhalb von ca. 5 Minuten zu. Es entsteht eine dünne Suspension, welche man mit 3,5 l Äthanol verdünnt und 16 Stunden am Rückfluss siedet. Die Suspension wird dann gekühlt, mit 2 l Wasser versetzt und zur Klärung mit einem Filterhilfsmittel (HiFlo) versetzt und filtriert. Dann gibt man konzentrierte Salzsäure dazu bis zum Kongo-blau Umschlag und lässt einige Stunden in der Kühle stehen. Es bildet sich ein feiner weisser, kristalliner Niederschlag, den man filtriert und trocknet. Die Mutterlauge wird eingeengt, mit Wasser verdünnt und der Niederschlag wird ebenfalls filtriert und getrocknet. Die Rohausbeute beträgt 263 g Material, welches bei 200 - 215° schmilzt. Nach Umkristallisieren aus Wasser verbleiben 144 g weisse Kristalle (58 % der Theorie), welche bei 203 - 204° schmelzen.

b) Herstellung von 5-Methyl-2,4,6-trichlorpyrimidin

In einem 2 l Kolben legt man 450 ml (2,94 Mol) Phosphoroxychlorid vor und gibt langsam unter Kühlen und Rühren 49,3 ml (0,39 Mol) N,N-Dimethylanilin techn. dazu. Weiter gibt man portionenweise innert 20 Minuten 142 g (1 Mol) Methylbarbitursäure zu, was eine exotherme Reaktion bewirkt. Man lässt das Reaktionsgemisch sich erwärmen und kocht schliesslich noch 5 Stunden am Rückfluss, bis die Gasentwicklung aufgehört hat Dann giesst man die Suspension langsam auf Wasser, wobei man darauf achtet, dass die Temperatur bei 25 -

30° bleibt. Die wässrige Suspension wird abgenutscht, das Nutschgut wird in 250 ml Dichlormethan gelöst, zur Klärung filtriert und 2 mal mit je 250 ml Eiswasser extrahiert. Dann wird die organische Phase getrocknet, eingeengt und der kristallin anfallende Rückstand im Vakuum getrocknet. Es verbleiben 126,7 g kristallines 5-Methyl-2,4,6-trichlorpyrimidin, welches bei 67 - 68° schmilzt (Ausbeute = 64,2 % der Theorie).

c) Herstellung von 4-Amino-2,6-dichlor-5-methylpyrimidin

Man löst 39,5 g (0,2 Mol) 5-Methyl-2,4,6-trichlor-pyrimidin in 250 ml Dimethoxyäthan und leitet dann bei Raumtemperatur Ammoniak-Gas bis zur Sättigung in die Lösung. Es entsteht eine weisse Suspension, die man während 12 Stunden bei Raumtemperatur rührt. Dann wird am Rotationsverdampfer eingeengt und der Rückstand mit 200 ml Wasser versetzt. Die entstandene Suspension wird filtriert und der Niederschlag getrocknet. Die Mutterlauge wird auf das halbe Volumen eingeengt, filtriert und der Niederschlag getrocknet. Man erhält so 26,4 g Rohprodukt, welches aus Acetonitril/Wasser 5 : 1 umkristallisiert wird. Schmelzpunkt 193 - 194° (Ausbeute = 75 % der Theorie).

| | | | | |
|---|---|---|---|---|
| Analyse: | ber. C 33,74 % | H 2,83 % | N 23,61 % | Cl 39,83 % |
| | gef. C 33,80 % | H 2,87 % | N 23,44 % | Cl 39,51 %. |

d) Herstellung von 4-Amino-2,6-dimethoxy-5-methylpyrimidin

Man lässt im Autoklaven bei 120° während 20 Stunden ein Gemisch von 10,7 g (0,06 Mol) 5-Methyl-4-amino-2,6-dichlorpyrimidin, 100 ml Methanol und 22 g 30 %-iges Natrium-Methylat (0,122 Mol) reagieren. Nach dem Abkühlen wird das graue Reaktionsgemisch am Vakuum eingeengt, der Rückstand in 70 ml Wasser aufgenommen, verrührt und filtriert. Der Rückstand wird mit Wasser gewaschen, getrocknet und aus Tetrachlorkohlenstoff umkristallisiert. Nach dem Trocknen verbleiben 6,1 g 4-Amino-5-methyl-2,6-dimethoxypyrimidin als weisse Kristalle, welche bei 60° schmelzen (Ausbeute = 60 % der Theorie).

| | | | | |
|---|---|---|---|---|
| Analyse | ber. C 49,70 % | H 6,55 % | N 24,84 % | O 18,91 % |
| | gef. C 49,45 % | H 6,42 % | N 24,59 % | O 19,84 %. |

In analoger Weise zu diesem Beispiel werden folgende Verbindungen erhalten:

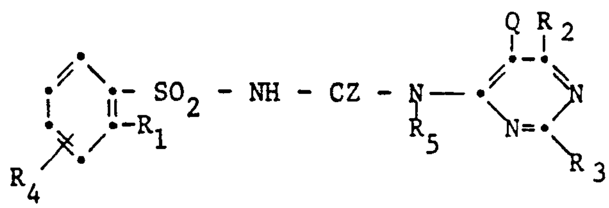

**Tabelle 1:**

| No. | Q | $R_1$ | $R_2$ | $R_3$ | $R_4$ | $R_5$ | Z | phys.Daten Smp. |
|---|---|---|---|---|---|---|---|---|
| 1.1 | $CH_3$ | $COOCH_3$ | $CH_3$ | $OCH_3$ | H | H | O | |
| 1.2 | $CH_3$ | $COOCH_3$ | $OCH_3$ | $OCH_3$ | H | H | O | 195 - 196° |
| 1.3 | $CH_3$ | $COOCH_3$ | $C_2H_5$ | $OCH_3$ | H | H | O | |
| 1.4 | $CH_3$ | $COOCH_3$ | $N(CH_3)_2$ | $OCH_3$ | H | H | O | |
| 1.5 | $CH_3$ | $COOCH_3$ | $CH_2F$ | $OCH_3$ | H | H | O | |
| 1.6 | $CH_3$ | $COOCH_3$ | Cyclopropyl | $OCH_3$ | H | H | O | |
| 1.7 | $CH_2$ | $COOCH_3$ | $SCH_3$ | $OCH_3$ | H | H | O | |
| 1.8 | $CH_3$ | $COOCH_3$ | $CH_2OCH_3$ | $OCH_3$ | H | H | O | |
| 1.9 | $CH_3$ | $COOCH_3$ | $CH_2OC_2H_5$ | $OCH_3$ | H | H | O | |
| 1.10 | $CH_3$ | $COOCH_3$ | $NHCH_3$ | $OCH_3$ | H | H | O | |
| 1.11 | $CH_3$ | $COOCH_3$ | $NH_2$ | $OCH_3$ | H | H | O | |
| 1.12 | $CH_3$ | $COOCH_3$ | $OCH_2CF_3$ | $OCH_3$ | H | H | O | 158 - 159° |
| 1.13 | $CH_3$ | $COOCH_3$ | $OCHF_2$ | $OCH_3$ | H | H | O | |
| 1.14 | $CH_3$ | $COOCH_3$ | $OC_2H_4OCH_3$ | $OCH_3$ | H | H | O | |
| 1.15 | $CH_3$ | $COOCH_3$ | Cl | $OCH_3$ | H | H | O | |
| 1.16 | $CH_3$ | $COOCH_3$ | $OC_2H_5$ | $OCH_3$ | H | H | O | |
| 1.17 | Cl | $COOCH_3$ | $CH_3$ | $OCH_3$ | H | H | O | |
| 1.18 | Cl | $COOCH_3$ | $OCH_3$ | $OCH_3$ | H | H | O | 188 - 189° |
| 1.19 | Cl | $COOCH_3$ | $C_2H_5$ | $OCH_3$ | H | H | O | |
| 1.20 | Cl | $COOCH_3$ | $N(CH_3)$ | $OCH_3$ | H | H | O | 192 - 194° |

| | | | | | | | | |
|---|---|---|---|---|---|---|---|---|
| 1.21 | Cl | $COOCH_3$ | $CH_2F$ | $OCH_3$ | H | H | O | |
| 1.22 | Cl | $COOCH_3$ | Cyclopropyl | $OCH_3$ | H | H | O | |
| 1.23 | Cl | $COOCH_3$ | $SCH_3$ | $OCH_3$ | H | H | 0 | |
| 1.24 | Cl | $COOCH_3$ | $CH_2OCH_3$ | $OCH_3$ | H | H | O | |
| 1.25 | Cl | $COOCH_3$ | $CH_2OC_2H_5$ | $OCH_3$ | H | H | O | |
| 1.26 | Cl | $COOCH_3$ | $NHCH_3$ | $OCH_3$ | H | H | O | |
| 1.27 | Cl | $COOCH_3$ | $NH_2$ | $OCH_3$ | H | H | O | |
| 1.28 | Cl | $COOCH_3$ | $OCH_2CF_3$ | $OCH_3$ | H | H | O | |
| 1.29 | Cl | $COOCH_3$ | $OCHF_2$ | $OCH_3$ | H | H | O | |
| 1.30 | Cl | $COOCH_3$ | $OC_2H_4OCH_3$ | $OCH_3$ | H | H | O | 198 - 200 |
| 1.31 | Cl | $COOCH_3$ | Cl | $OCH_3$ | H | H | O | |
| 1.32 | Cl | $COOCH_3$ | $OC_2H_5$ | $OCH_3$ | H | H | O | |
| 1.33 | $CH_3$ | $OCHF_2$ | $CH_3$ | $OCH_3$ | H | H | O | |
| 1.34 | $CH_3$ | $OCHF_2$ | $OCH_3$ | $OCH_3$ | H | H | O | 184 - 185 |
| 1.35 | $CH_3$ | $OCHF_2$ | $C_2H_5$ | $OCH_3$ | H | H | O | |
| 1.36 | $CH_3$ | $OCHF_2$ | $N(CH_3)_2$ | $OCH_3$ | H | H | O | |
| 1.37 | $CH_3$ | $OCHF_2$ | $CH_2F$ | $OCH_3$ | H | H | O | |
| 1.38 | $CH_3$ | $OCHF_2$ | Cyclopropyl | $OCH_3$ | H | H | O | |
| 1.39 | $CH_3$ | $OCHF_2$ | $SCH_3$ | $OCH_3$ | H | H | O | |
| 1.40 | $CH_3$ | $OCHF_2$ | $CH_2OCH_3$ | $OCH_3$ | H | H | O | |
| 1.41 | $CH_3$ | $OCHF_2$ | $CH_2OC_2H_5$ | $OCH_3$ | H | H | O | |
| 1.42 | $CH_3$ | $OCHF_2$ | $NHCH_3$ | $OCH_3$ | H | H | O | |
| 1.43 | $CH_3$ | $OCHF_2$ | $NH_2$ | $OCH_3$ | H | H | O | |
| 1.44 | $CH_3$ | $OCHF_2$ | $OCH_2CF_3$ | $OCH_3$ | H | H | O | |
| 1.45 | $CH_3$ | $OCHF_2$ | $OCHF_2$ | $OCH_3$ | H | H | O | |
| 1.46 | $CH_3$ | $OCHF_2$ | $OC_2H_4OCH_3$ | $OCH_3$ | H | H | O | |
| 1.47 | $CH_3$ | $OCHF_2$ | Cl | $OCH_3$ | H | H | O | |
| 1.48 | $CH_3$ | $OCHF_2$ | $OC_2H_5$ | $OCH_3$ | H | H | O | |
| 1.49 | Cl | $OCHF_2$ | $CH_3$ | $OCH_3$ | H | H | O | 177 - 178 |
| 1.50 | Cl | $OCHF_2$ | $OCH_3$ | $OCH_3$ | H | H | O | 190 - 191 |
| 1.51 | Cl | $OCHF_2$ | $C_2H_5$ | $OCH_3$ | H | H | O | |
| 1.52 | Cl | $OCHF_2$ | $N(CH_3)_2$ | $OCH_3$ | H | H | O | 169 - 170 |
| 1.53 | Cl | $OCHF_2$ | $CH_2F$ | $OCH_3$ | H | H | O | |
| 1.54 | Cl | $OCHF_2$ | Cyclopropyl | $OCH_3$ | H | H | O | |
| 1.55 | Cl | $OCHF_2$ | $SCH_3$ | $OCH_3$ | H | H | O | |
| 1.56 | Cl | $OCHF_2$ | $CH_2OCH_3$ | $OCH_3$ | H | H | O | |
| 1.57 | Cl | $OCHF_2$ | $CH_2OC_2H_5$ | $OCH_3$ | H | H | O | |
| 1.58 | Cl | $OCHF_2$ | $NHCH_3$ | $OCH_3$ | H | H | O | |
| 1.59 | Cl | $OCHF_2$ | $NH_2$ | $OCH_3$ | H | H | O | |
| 1.60 | Cl | $OCHF_2$ | $OCH_2CF_3$ | $OCH_3$ | H | H | O | |
| 1.61 | Cl | $OCHF_2$ | $OCHF_2$ | $OCH_3$ | H | H | O | |
| 1.62 | Cl | $OCHF_2$ | $OC_2H_4OCH_3$ | $OCH_3$ | H | H | O | |
| 1.63 | Cl | $OCHF_2$ | Cl | $OCH_3$ | H | H | O | |
| 1.64 | Cl | $OCHF_2$ | $OC_2H_5$ | $OCH_3$ | H | H | O | |
| 1.65 | $CH_3$ | $CF_3$ | $OCH_3$ | $CH_3$ | H | H | O | |
| 1.66 | $CH_3$ | $CF_3$ | $OCH_3$ | $C_2H_5$ | H | H | O | |
| 1.67 | $CH_3$ | $CF_3$ | $OCH_3$ | $SCH_3$ | H | H | O | |
| 1.68 | $CH_3$ | $CF_3$ | $OCH_3$ | $OC_2H_5$ | H | H | O | |
| 1.69 | $CH_3$ | $CF_3$ | $OCH_3$ | $N(CH_3)_2$ | H | H | O | |
| 1.70 | $CH_3$ | $CF_3$ | $CH_3$ | $OC_2H_5$ | H | H | O | |
| 1.71 | $CH_3$ | $CF_3$ | $OCH_2CH_3$ | $OC_2H_5$ | H | H | O | |
| 1.72 | $CH_3$ | $CF_3$ | $CH_3$ | $CH_3$ | H | H | O | |
| 1.73 | Cl | $CF_3$ | $OCH_3$ | $CH_3$ | H | H | O | |
| 1.74 | Cl | $CF_3$ | $OCH_3$ | $C_2H_5$ | H | H | O | |
| 1.75 | Cl | $CF_3$ | $OCH_3$ | $SCH_3$ | H | H | O | |
| 1.76 | Cl | $CF_3$ | $OCH_3$ | $OC_2H_5$ | H | H | O | |
| 1.77 | Cl | $CF_3$ | $OCH_3$ | $N(CH_3)_2$ | H | H | O | |
| 1.78 | Cl | $CF_3$ | $CH_3$ | $OC_2H_5$ | H | H | O | |
| 1.79 | Cl | $CF_2$ | $OCH_2CH_3$ | $OC_2H_5$ | H | H | O | |
| 1.80 | Cl | $CF_3$ | $OCH_3$ | $OCH_2CF_3$ | H | H | O | |
| 1.81 | Cl | $CF_3$ | $CH_3$ | $CH_3$ | H | H | O | |
| 1.82 | $CH_3$ | $NO_2$ | $OCH_3$ | $CH_3$ | H | H | O | |
| 1.83 | $CH_3$ | $NO_2$ | $OCH_3$ | $C_2H_5$ | H | H | O | |
| 1.84 | $CH_3$ | $NO_2$ | $OCH_3$ | $SCH_3$ | H | H | O | |
| 1.85 | $CH_3$ | $NO_2$ | $OCH_3$ | $OC_2H_5$ | H | H | O | |

| | | | | | | | | |
|---|---|---|---|---|---|---|---|---|
| 1.86 | $CH_3$ | $NO_2$ | $OCH_3$ | $N(CH_3)_2$ | H | H | O | |
| 1.87 | $CH_3$ | $NO_2$ | $CH_3$ | $OC_2H_5$ | H | H | O | |
| 1.88 | $CH_3$ | $NO_2$ | $OCH_2CH_3$ | $OC_2H_5$ | H | H | O | |
| 1.89 | $CH_3$ | $NO_2$ | $CH_3$ | $CH_3$ | H | H | O | |
| 1.90 | $CH_3$ | $NO_2$ | $OCH_3$ | $OCH_2CF_3$ | H | H | O | |
| 1.91 | Cl | $NO_2$ | $OCH_3$ | $CH_3$ | H | H | O | |
| 1.92 | Cl | $NO_2$ | $OCH_3$ | $C_2H_5$ | H | H | O | |
| 1.93 | Cl | $NO_2$ | $OCH_3$ | $SCH_3$ | H | H | O | |
| 1.94 | Cl | $NO_2$ | $OCH_3$ | $OC_2H_5$ | H | H | O | |
| 1.95 | Cl | $NO_2$ | $OCH_3$ | $N(CH_3)_2$ | H | H | O | |
| 1.96 | Cl | $NO_2$ | $CH_3$ | $OC_2H_5$ | H | H | O | |
| 1.97 | Cl | $NO_2$ | $OCH_2CH_3$ | $OC_2H_5$ | H | H | O | |
| 1.98 | Cl | $NO_2$ | $OCH_3$ | $OCH_2CF_3$ | H | H | O | |
| 1.99 | Cl | $NO_2$ | $CH_3$ | $CH_3$ | H | H | O | |
| 1.100 | $CH_3$ | $OCHF_2$ | $OCH_3$ | $CH_3$ | H | H | O | |
| 1.101 | $CH_3$ | $OCHF_2$ | $OCH_3$ | $C_2H_5$ | H | H | O | |
| 1.102 | $CH_3$ | $OCHF_2$ | $OCH_3$ | $SCH_3$ | H | H | O | |
| 1.103 | $CH_3$ | $OCHF_2$ | $OCH_3$ | $OC_2H_5$ | H | H | O | |
| 1.104 | $CH_3$ | $OCHF_2$ | $OCH_3$ | $N(CH_3)_2$ | H | H | O | |
| 1.105 | $CH_3$ | $OCHF_2$ | $CH_3$ | $OC_2H_5$ | H | H | O | |
| 1.106 | $CH_3$ | $OCHF_2$ | $OCH_2CH_3$ | $OC_2H_5$ | H | H | O | |
| 1.107 | $CH_3$ | $OCHF_2$ | $CH_3$ | $CH_3$ | H | H | O | |
| 1.108 | $CH_3$ | $OCHF_2$ | $OCH_3$ | $OCH_2CF_3$ | H | H | O | |
| 1.109 | $CH_3$ | $COOCH_3$ | $OCH_3$ | $CH_3$ | H | H | O | |
| 1.110 | $CH_3$ | $COOCH_3$ | $OCH_3$ | $C_2H_5$ | H | H | O | 182 - 183 |
| 1.111 | $CH_3$ | $COOCH_3$ | $OCH_3$ | $OC_2H_5$ | H | H | O | |
| 1.112 | $CH_3$ | $COOCH_3$ | $OCH_3$ | $N(CH_3)_2$ | H | H | O | |
| 1.113 | $CH_3$ | $COOCH_3$ | $CH_3$ | $OC_2H_5$ | H | H | O | |
| 1.114 | $CH_3$ | $COOCH_3$ | $OCH_2CH_3$ | $OC_2H_5$ | H | H | O | |
| 1.115 | $CH_3$ | $COOCH_3$ | $OCH_3$ | $OCH_2CF_3$ | H | H | O | |
| 1.116 | $CH_3$ | $COOCH_3$ | $CH_3$ | $CH_3$ | H | H | O | |
| 1.117 | $CH_3$ | $COOCH_3$ | Cl | $C_2H_5$ | H | H | O | 199 - 200 |
| 1.118 | Cl | $OCHF_2$ | $OCH_3$ | $CH_3$ | H | H | O | 212 - 213 |
| 1.119 | Cl | $OCHF_2$ | $OCH_3$ | $C_2H_5$ | H | H | O | |
| 1.120 | Cl | $OCHF_2$ | $OCH_3$ | $SCH_3$ | H | H | O | |
| 1.121 | Cl | $OCHF_2$ | $OCH_3$ | $OC_2H_5$ | H | H | O | |
| 1.122 | Cl | $OCHF_2$ | $OCH_3$ | $N(CH_3)_2$ | H | H | O | 218 - 219 |
| 1.123 | Cl | $OCHF_2$ | $CH_3$ | $OC_2H_5$ | H | H | O | |
| 1.124 | Cl | $OCHF_2$ | $OCH_2CH_3$ | $OC_2H_5$ | H | H | O | |
| 1.125 | Cl | $OCHF_2$ | $CH_3$ | $CH_3$ | H | H | O | |
| 1.126 | Cl | $OCHF_2$ | $OCH_3$ | $OCH_2CF_3$ | H | H | O | |
| 1.127 | Cl | $COOCH_3$ | $OCH_3$ | $CH_3$ | H | H | O | |
| 1.128 | Cl | $COOCH_3$ | $OCH_3$ | $C_2H_5$ | H | H | O | |
| 1.129 | Cl | $COOCH_3$ | $OCH_3$ | $SCH_3$ | H | H | O | |
| 1.130 | Cl | $COOCH_3$ | $OCH_3$ | $N(CH_3)_2$ | H | H | O | |
| 1.131 | Cl | $COOCH_3$ | $CH_3$ | $OC_2H_5$ | H | H | O | |
| 1.132 | Cl | $COOCH_3$ | $OCH_2CH_3$ | $OC_2H_5$ | H | H | O | |
| 1.133 | Cl | $COOCH_3$ | $OCH_3$ | $OC_2H_5$ | H | H | O | |
| 1.134 | Cl | $COOCH_3$ | $CH_3$ | $CH_3$ | H | H | O | 174 - 175 |
| 1.135 | Cl | $COOCH_3$ | $OCH_3$ | Cl | H | H | O | 215 - 218 |
| 1.136 | F | $COOCH_3$ | $CH_3$ | $OCH_3$ | H | H | O | |
| 1.137 | F | $COOCH_3$ | $OCH_3$ | $OCH_3$ | H | H | O | 181 - 182 |
| 1.138 | F | $COOCH_3$ | $N(CH_3)_2$ | $OCH_3$ | H | H | O | |
| 1.139 | F | $COOCH_3$ | $C_2H_5$ | $OCH_3$ | H | H | O | |
| 1.140 | $NH_2$ | $COOCH_3$ | $CH_3$ | $OCH_3$ | H | H | O | |
| 1.141 | $NH_2$ | $COOCH_3$ | $OCH_3$ | $OCH_3$ | H | H | O | |
| 1.142 | $NH_2$ | $COOCH_3$ | $N(CH_3)_2$ | $OCH_3$ | H | H | O | |
| 1.143 | $NH_2$ | $COOCH_3$ | $C_2H_5$ | $OCH_3$ | H | H | O | |
| 1.144 | $CF_3$ | $COOCH_3$ | $CH_3$ | $OCH_3$ | H | H | O | |
| 1.145 | $CF_3$ | $COOCH_3$ | $OCH_3$ | $OCH_3$ | H | H | O | |
| 1.146 | $CF_3$ | $COOCH_3$ | $N(CH_3)_2$ | $OCH_3$ | H | H | O | |
| 1.147 | $CF_3$ | $COOCH_3$ | $C_2H_5$ | $OCH_3$ | H | H | O | |
| 1.148 | $CF_3$ | $CCOCH_3$ | F | $N(CH_3)_2$ | H | H | O | 157 - 158 |
| 1.149 | $CF_3$ | $COOCH_3$ | F | $NHCH_3$ | H | H | O | 135 - 137 |
| 1.150 | $C_2H_5$ | $COOCH_3$ | $CH_3$ | $OCH_3$ | H | H | O | |

| | | | | | | | | |
|---|---|---|---|---|---|---|---|---|
| 1.151 | $C_2H_5$ | $COOCH_3$ | $OCH_3$ | $OCH_3$ | H | H | O | |
| 1.152 | $C_2H_5$ | $COOCH_3$ | $N(CH_3)_2$ | $OCH_3$ | H | H | O | |
| 1.153 | $C_2H_5$ | $COOCH_3$ | $C_2H_5$ | $OCH_3$ | H | H | O | |
| 1.154 | $OCH_3$ | $COOCH_3$ | $CH_3$ | $OCH_3$ | H | H | O | |
| 1.155 | $OCH_3$ | $COOCH_3$ | $OCH_3$ | $OCH_3$ | H | H | O | |
| 1.156 | $OCH_3$ | $COOCH_3$ | $N(CH_3)_2$ | $OCH_3$ | H | H | O | |
| 1.157 | $OCH_3$ | $COOCH_3$ | $C_2H_5$ | $OCH_3$ | H | H | O | |
| 1.158 | CHO | $COOCH_3$ | $CH_3$ | $OCH_3$ | H | H | O | |
| 1.159 | CHO | $COOCH_3$ | $OCH_3$ | $OCH_3$ | H | H | O | 184 - 185° |
| 1.160 | CHO | $COOCH_3$ | $N(CH_3)_2$ | $OCH_3$ | H | H | O | |
| 1.161 | CHO | $COOCH_3$ | $C_2H_5$ | $OCH_3$ | H | H | O | |
| 1.162 | $CH_3$ | $NO_2$ | $CH_3$ | $OCH_3$ | H | H | O | |
| 1.163 | $CH_3$ | $NO_2$ | $OCH_3$ | $OCH_3$ | H | H | O | 202 - 203° |
| 1.164 | $CH_3$ | $NO_2$ | $N(CH_3)_2$ | $OCH_3$ | H | H | O | |
| 1.165 | $CH_3$ | $NO_2$ | $C_2H_5$ | $OCH_3$ | H | H | O | |
| 1.166 | Cl | $NO_2$ | $CH_3$ | $OCH_3$ | H | H | O | |
| 1.167 | Cl | $NO_2$ | $OCH_3$ | $OCH_3$ | H | H | O | |
| 1.168 | Cl | $NO_2$ | $N(CH_3)_2$ | $OCH_3$ | H | H | O | 185 - 186° |
| 1.169 | Cl | $NO_2$ | $C_2H_5$ | $OCH_3$ | H | H | O | |
| 1.170 | $CH_3$ | $CF_3$ | $CH_3$ | $OCH_3$ | H | H | O | |
| 1.171 | $CH_3$ | $CF_3$ | $OCH_3$ | $OCH_3$ | H | H | O | |
| 1.172 | $CH_3$ | $CF_3$ | $N(CH_3)_2$ | $OCH_3$ | H | H | O | |
| 1.173 | $CH_3$ | $CF_3$ | $C_2H_5$ | $OCH_3$ | H | H | O | |
| 1.174 | Cl | $CF_3$ | $CH_3$ | $OCH_3$ | H | H | O | |
| 1.175 | Cl | $CF_3$ | $OCH_3$ | $OCH_3$ | H | H | O | |
| 1.176 | Cl | $CF_2$ | $N(CH_3)_2$ | $OCH_3$ | H | H | O | |
| 1.177 | Cl | $CF_3$ | $C_2H_5$ | $OCH_3$ | H | H | O | |
| 1.178 | $SCH_3$ | $COOCH_3$ | $CH_3$ | $OCH_3$ | H | H | O | |
| 1.179 | $SCH_3$ | $COOCH_3$ | $OCH_3$ | $OCH_3$ | H | H | O | |
| 1.180 | $SCH_3$ | $COOCH_3$ | $N(CH_3)_2$ | $OCH_3$ | H | H | O | |
| 1.181 | $SCH_3$ | $COOCH_3$ | $C_2H_5$ | $OCH_3$ | H | H | O | |
| 1.182 | $SOCH_3$ | $COOCH_3$ | $CH_3$ | $OCH_3$ | H | H | O | |
| 1.183 | $SOCH_3$ | $COOCH_3$ | $OCH_3$ | $OCH_3$ | H | H | O | |
| 1.184 | $SOCH_3$ | $COOCH_3$ | $N(CH_3)_2$ | $OCH_3$ | H | H | O | |
| 1.185 | $SOCH_3$ | $COOCH_3$ | $C_2H_5$ | $OCH_3$ | H | H | O | |
| 1.186 | $CH_3$ | $COSCH_3$ | $OCH_3$ | $OCH_3$ | H | H | O | |
| 1.187 | $CH_3$ | $CH_2OCH_3$ | $OCH_3$ | $OCH_3$ | H | H | O | |
| 1.188 | $CH_3$ | $CH_2SCH_3$ | $OCH_3$ | $OCH_3$ | H | H | O | |
| 1.189 | $CH_3$ | $CH_2SOCH_3$ | $OCH_3$ | $OCH_3$ | H | H | O | |
| 1.190 | $CH_3$ | $CH_2SO_2CH_3$ | $OCH_3$ | $OCH_3$ | H | H | O | |
| 1.191 | $CH_3$ | $C_2H_4OCH_3$ | $OCH_3$ | $OCH_3$ | H | H | O | |
| 1.192 | $CH_3$ | $SCH_2CH=CH_2$ | $OCH_3$ | $OCH_3$ | H | H | O | |
| 1.193 | $CH_3$ | $OCH_2CCl=CH_2$ | $OCH_3$ | $OCH_3$ | H | H | O | |
| 1.194 | $CH_3$ | $CH=CHCN$ | $OCH_3$ | $OCH_3$ | H | H | O | |
| 1.195 | $CH_3$ | $C_3H_6OCH_3$ | $OCH_3$ | $OCH_3$ | H | H | O | |
| 1.196 | $CH_3$ | $CH_2COCH_3$ | $OCH_3$ | $OCH_3$ | H | H | O | |
| 1.197 | $CH_3$ | $OCH_3$ | $OCH_3$ | $OCH_3$ | H | H | O | |
| 1.198 | $CH_3$ | $OC_2H_5$ | $OCH_3$ | $OCH_3$ | H | H | O | 216 - 217° |
| 1.199 | $CH_3$ | $OC_2H_4OCH_3$ | $OCH_3$ | $OCH_3$ | H | H | O | |
| 1.200 | $CH_3$ | $OCH_2CH=CH_2$ | $OCH_3$ | $OCH_3$ | H | H | O | |
| 1.201 | $CH_3$ | $OCH_2CH=CH$ | $OCH_3$ | $OCH_3$ | H | H | O | |
| 1.202 | $CH_3$ | $OCF_3$ | $OCH_3$ | $OCH_3$ | H | H | O | 189 - 190° |
| 1.202 | $CH_3$ | $OCF_2CHF_2$ | $OCH_3$ | $OCH_3$ | H | H | O | |
| 1.203 | $CH_3$ | $OCF_2CF_3$ | $OCH_3$ | $OCH_3$ | H | H | 0 | |
| 1.204 | $CH_3$ | $OCF_2CF_3$ | $OCH_3$ | $OCH_3$ | H | H | O | |
| 1.205 | $CH_3$ | $OCH_2CH_2Cl$ | $OCH_3$ | $OCH_3$ | H | H | O | 214 - 215 |
| 1.206 | $CH_3$ | $OCHCl=CHCl$ | $OCH_3$ | $OCH_3$ | H | H | O | 222 - 223° |
| 1.207 | $CH_3$ | $CH_3$ | $OCH_3$ | $OCH_3$ | H | H | O | |
| 1.208 | $CH_3$ | $C_2H_5$ | $OCH_3$ | $OCH_3$ | H | H | O | |
| 1.209 | $CH_3$ | $nC_3H_7$ | $OCH_3$ | $OCH_3$ | H | H | O | |
| 1.210 | $CH_3$ | $SCF_3$ | $OCH_3$ | $OCH_3$ | H | H | O | |
| 1.211 | $CH_3$ | $CH_2Cl$ | $OCH_3$ | $OCH_3$ | H | H | O | |
| 1.212 | $CH_3$ | $C_2H_4CF_3$ | $OCH_3$ | $OCH_3$ | H | H | O | |
| 1.213 | $CH_3$ | Br | $OCH_3$ | $OCH_3$ | H | H | O | |
| 1.214 | $CH_3$ | F | $OCH_3$ | $OCH_3$ | H | H | O | |

| | | | | | | | | |
|---|---|---|---|---|---|---|---|---|
| 1.215 | $CH_3$ | $SCH_3$ | $OCH_3$ | $OCH_3$ | H | H | O | |
| 1.216 | $CH_3$ | $SOCH_3$ | $OCH_3$ | $OCH_3$ | H | H | O | |
| 1.217 | $CH_3$ | $SO_2CH_3$ | $OCH_3$ | $OCH_3$ | H | H | O | |
| 1.218 | $CH_3$ | $SO_2C_3H_7n$ | $OCH_3$ | $OCH_3$ | H | H | O | |
| 1.219 | $CH_3$ | $SO_2N(CH_3)_2$ | $OCH_3$ | $OCH_3$ | H | H | O | |
| 1.229 | $CH_3$ | CHO | $OCH_3$ | $OCH_3$ | H | H | O | |
| 1.221 | $CH_3$ | $COCH_3$ | $OCH_3$ | $OCH_3$ | H | H | O | |
| 1.222 | $CH_3$ | $COOC_2H_5$ | $OCH_3$ | $OCH_3$ | H | H | O | |
| 1.223 | CH | $COOCH_2CH=CH_2$ | $OCH_3$ | $OCH_3$ | H | H | O | |
| 1.224 | $CH_3$ | $OSO_2CH_3$ | $OCH_3$ | $OCH_3$ | H | H | O | |
| 1.225 | $CH_3$ | $OSO_2CF_3$ | $OCH_3$ | $OCH_3$ | H | H | O | |
| 1.226 | $CH_3$ | $COCF_3$ | $OCH_3$ | $OCH_3$ | H | H | O | |
| 1.227 | $CH_3$ | $CH=CH_2$ | $OCH_3$ | $OCH_3$ | H | H | O | |
| 1.228 | $CH_3$ | $CH=CHCH_3$ | $OCH_3$ | $OCH_3$ | H | H | O | |
| 1.229 | $CH_3$ | $CH=CCl_2$ | $OCH_3$ | $OCH_3$ | H | H | O | |
| 1.230 | $CH_3$ | $CH=CHCF_3$ | $OCH_3$ | $OCH_3$ | H | H | O | |
| 1.231 | $CH_3$ | $CH_2$ | $OCH_3$ | $OCH_3$ | H | H | O | |
| 1.232 | $CH_3$ | $CON(CH_3)_2$ | $OCH_3$ | $OCH_3$ | H | H | O | |
| 1.233 | Cl | $OCH_3$ | $OCH_3$ | $OCH_3$ | 5-$OCH_3$ | H | O | 201 - 203° |
| 1.234 | $SOCH_3$ | Cl | $OCH_3$ | $OCH_3$ | H | H | O | 161 - 165° |
| 1.235 | Cl | $SCHF_2$ | $OCH_3$ | $OCH_3$ | H | H | O | 184 - 185° |
| 1.236 | F | $OCHF_2$ | $OCH_3$ | $OCH_3$ | H | H | O | 156 - 157° |
| 1.237 | F | Cl | $OCH_3$ | $OCH_3$ | H | H | O | 216 - 217° |
| 1.238 | Br | $COOCH_3$ | $CH_3$ | $OCH_3$ | H | H | O | 177 - 178° |
| 1.239 | $NH_2$ | Cl | $CH_3$ | $OCH_3$ | H | H | O | 175 - 178° |
| 1.240 | $CH_3$ | $COOCH_3$ | $OCH_3$ | $OCH_3$ | H | $CH_3$ | O | |
| 1.241 | $CH_3$ | $COOCH_3$ | $OCH_3$ | $OCH_3$ | H | H | S | |
| 1.242 | $CH_3$ | Cl | $OCH_3$ | $OCH_3$ | H | H | S | |
| 1.243 | $CH_3$ | $OCHF_2$ | $OCH_3$ | $OCH_3$ | H | H | S | |
| 1.244 | $CH_3$ | $OCHF_2$ | $OCH_3$ | $OCH_3$ | H | $CH_3$ | O | |
| 1.245 | $CH_3$ | $COOCH_3$ | $CH_3$ | $OCH_3$ | H | $CH_3$ | O | |
| 1.246 | Cl | $OC_2H_4OCH_3$ | $N(CH_3)_2$ | $OCH_3$ | H | H | O | 165 - 166° |
| 1.247 | $CH_3$ | Cl | $OCH_3$ | $OCH_3$ | H | H | O | 221 - 222° |
| 1.248 | F | $COOCH_3$ | H | $OCH_3$ | H | H | O | 196 - 197° |
| 1.249 | Cl | $COOCH_3$ | H | $OCH_3$ | H | H | O | 190 - 192° |
| 1.250 | $CH_2CH_3$ | $COOCH_3$ | OCHF | $C_3H_7(n)$ | H | H | O | 170 - 171° |
| 1.251 | $CH_3$ | $COOCH_3$ | $OCH_3$ | $CH_2OCH_3$ | H | H | O | 170 - 171° |
| 1.152 | $C_3H_7(i)$ | $COOCH_3$ | $OCH_3$ | $OCH_3$ | H | H | O | 170 - 171° |
| 1.253 | $CH_3$ | $COOCH_3$ | Cl | $CH_2OCH_3$ | H | H | O | 193 - 194° |
| 1.254 | $CH_3$ | $COOCH_3$ | $OCH_2CH_3$ | $CH_2OCH_3$ | H | H | O | 163 - 164° |
| 1.255 | $SCH_3$ | $COOCH_3$ | $OCH_3$ | $C_3H_7(i)$ | H | H | O | 146 - 147° |
| 1.256 | $SOCH_3$ | $COOCH_3$ | $OCH_3$ | $C_3H_7(i)$ | H | H | O | 167 - 168° |
| 1.257 | J | $COOCH_3$ | $OCH_3$ | $OCH_3$ | H | H | O | 175 - 176° |
| 1.258 | $-CH=CH_2$ | $COOCH_3$ | $OCH_3$ | $OCH_3$ | H | H | O | 144 - 145° |
| 1.259 | $-C\equiv CH$ | $COOCH_3$ | $OCH_3$ | $OCH_3$ | H | H | O | 146 - 149°Z |
| 1.260 | Cl | $OCH_2CH_2Cl$ | $OCH_3$ | $OCH_3$ | H | H | O | |
| 1.261 | Cl | $OC_2H_4OCH_3$ | $OCH_3$ | $OCH_3$ | H | H | O | |
| 1.262 | Cl | $OCH_3$ | $OCH_3$ | $OCH_3$ | H | H | O | |
| 1.263 | Cl | $OCH_2CH_3$ | $OCH_3$ | $OCH_3$ | H | H | O | |
| 1.264 | Cl | $OCCl=CHCl$ | $OCH_3$ | $OCH_3$ | H | H | O | |
| 1.265 | Cl | $SCH_2CH_2Cl$ | $OCH_3$ | $OCH_3$ | H | H | O | |
| 1.266 | Cl | $OCF_2CHF_2$ | $OCH_3$ | $OCH_3$ | H | H | O | |
| 1.267 | Cl | $OCF_2CF_3$ | $OCH_3$ | $OCH_3$ | H | H | O | |
| 1.268 | Cl | Cl | $OCH_3$ | $OCH_3$ | H | H | O | |
| 1.269 | Cl | F | $OCH_3$ | $OCH_3$ | H | H | O | |
| 1.270 | Cl | $CH=CH-CH_3$ | $OCH_3$ | $OCH_3$ | H | H | O | |
| 1.271 | Cl | $SO_2N(CH_3)_2$ | $OCH_3$ | $OCH_3$ | H | H | O | |
| 1.272 | Cl | $CON(CH_3)_2$ | $OCH_3$ | $OCH_3$ | H | H | O | |
| 1.273 | F | $OCH_2CH_2Cl$ | $OCH_3$ | $OCH_3$ | H | H | O | |
| 1.274 | F | $OC_2H_4OCH_3$ | $OCH_3$ | $OCH_3$ | H | H | O | |
| 1.275 | F | $OCH_3$ | $OCH_3$ | $OCH_3$ | H | H | O | |
| 1.276 | F | $OCH_2CH_3$ | $OCH_3$ | $OCH_3$ | H | H | O | |
| 1.277 | F | $OCCl=CHCl$ | $OCH_3$ | $OCH_3$ | H | H | O | |
| 1.278 | F | $NO_2$ | $OCH_3$ | $OCH_3$ | H | H | O | |
| 1.279 | F | $OCH_2-CH=CH_2$ | $OCH_3$ | $OCH_3$ | H | H | O | |

| | | | | | | | |
|---|---|---|---|---|---|---|---|
| 1.280 | Cl | $OCH_2$-$CH=CH_2$ | $OCH_3$ | $OCH_3$ | H | H | O |
| 1.281 | Cl | $OCH_2CF_3$ | $OCH_3$ | $OCH_3$ | H | H | O |
| 1.282 | Cl | $OCH_2CH_2Cl$ | $CH_3$ | $OCH_3$ | H | H | O |
| 1.283 | Cl | $OC_2H_4OCH_3$ | $CH_3$ | $OCH_3$ | H | H | O |
| 1.284 | Cl | $OCH_3$ | $CH_3$ | $OCH_3$ | H | H | O |
| 1.285 | Cl | $OCH_2CH_3$ | $CH_3$ | $OCH_3$ | H | H | O |
| 1.286 | Cl | $OCCl=CHCl$ | $CH_3$ | $OCH_3$ | H | H | O |
| 1.287 | Cl | $OCH_2$-$CH=CH_2$ | $CH_3$ | $OCH_3$ | H | H | O |
| 1.288 | Cl | Cl | $CH_3$ | $OCH_3$ | H | H | O |
| 1.289 | Cl | F | $CH_3$ | $OCH_3$ | H | H | O |
| 1.290 | Cl | $SCHF_2$ | $CH_3$ | $OCH_3$ | H | H | O |
| 1.291 | $NH_2$ | $OCHF_2$ | $OCH_3$ | $OCH_3$ | H | H | O |
| 1.292 | $NH_2$ | $OCHF_2$ | $CH_3$ | $OCH_3$ | H | H | O |
| 1.293 | $SOCH_3$ | $NO_2$ | $OCH_3$ | $OCH_3$ | H | H | O |
| 1.294 | $SOCH_3$ | $NO_2$ | $CH_3$ | $OCH_3$ | H | H | O |
| 1.295 | $SOCH_3$ | $OCHF_2$ | $OCH_3$ | $OCH_3$ | H | H | O |
| 1.296 | $SOCH_3$ | $OCHF_2$ | $CH_3$ | $OCH_3$ | H | H | O |

**Tabelle 2**

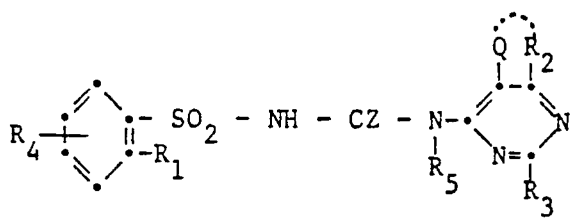

| No. | $R_1$ | -Q-$R_2$- | $R_3$ | $R_4$ | $R_5$ | Z |
|---|---|---|---|---|---|---|
| 2.1 | $COOCH_3$ | $-C_3H_6-$ | $OCH_3$ | H | H | O |
| 2.2 | $COOCH_3$ | $-C_4H_8-$ | $OCH_3$ | H | H | O |
| 2.3 | $COOCH_3$ | $-OC_3H_6-$ | $OCH_3$ | H | H | O |
| 2.4 | $COOCH_2$ | $-C_2H_4O-$ | $OCH_3$ | H | H | O |

**Tabelle 3**

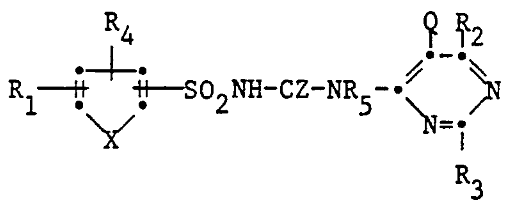

| No. | Q | $R_1$ | $R_2$ | $R_3$ | $R_4$ | $R_5$ | X | Z | Stellung des $SO_2$-Restes |
|---|---|---|---|---|---|---|---|---|---|
| 3.1 | $CH_3$ | 3-$COOCH_3$ | $OCH_3$ | $OCH_3$ | H | H | S | O | 2 |
| 3.2 | $CH_3$ | 2-$CHOOCH_3$ | $OCH_3$ | $OCH_3$ | H | H | S | O | 3 |
| 3.3 | $CH_3$ | 3-$COOCH_3$ | $OCH_3$ | $OCH_3$ | H | H | S | O | 4 |
| 3.4 | $CH_3$ | H | $OCH_3$ | $OCH_3$ | H | H | NH | O | 2 |
| 3.5 | $CH_3$ | H | $OCH_3$ | $OCH_3$ | H | H | NH | O | 3 |
| 3.6 | $CH_3$ | 2-Cl | $OCH_3$ | $OCH_3$ | H | H | -C=N- | O | 3 |
| 3.7 | $CH_3$ | 2-Cl | $CH_3$ | $OCH_3$ | H | H | -C=N- | O | 3 |
| 3.8 | $CH_3$ | 3-$COOCH_3$ | $CH_3$ | $OCH_3$ | H | H | -C=N- | O | 2 |
| 3.9 | $CH_3$ | 2-$COOCH_3$ | $CH_3$ | $OCH_3$ | H | H | -C=N- | O | 3 |
| 3.10 | $CH_3$ | 3-$COOCH_3$ | $CH_3$ | $OCH_3$ | H | H | -C=N- | O | 4 |
| 3.11 | $CH_3$ | 2-$OCH_2CH=CH_2$ | $CH_2$ | $OCH_3$ | H | H | -C=N- | O | 3 |
| 3.12 | $CH_3$ | 2-$OCH_2CH$-$CH_2$ | $OCH_3$ | $OCH_3$ | H | H | -C=N- | O | 3 |
| 3.13 | $CH_3$ | 2-$OCH_2CH_2CH_2$ | $CH_3$ | $OCH_3$ | H | H | -C=N- | O | 3 |
| 3.14 | $CH_3$ | 2-$OCH_3CH_2CH_3$ | $OCH_3$ | $OCH_3$ | H | H | -C=N- | O | 3 |
| 3.15 | $CH_3$ | 2-$OCH_3$ | $CH_3$ | $OCH_3$ | H | H | -C=N- | O | 3 |

| | | | | | | | | | |
|---|---|---|---|---|---|---|---|---|---|
| 3.16 | $CH_3$ | 2-$OCH_3$ | $OCH_3$ | $OCH_3$ | H | H | -C=N- | O | 3 |
| 3.17 | $CH_3$ | 2-$OCH_2CH=CHCH_3$ | $CH_3$ | $OCH_3$ | H | H | -C=N- | O | 3 |
| 3.18 | $CH_3$ | 2-$OCH_2CH=CHCH_3$ | $OCH_3$ | $OCH_3$ | H | H | -C=N- | O | 3 |

**Tabelle 4**

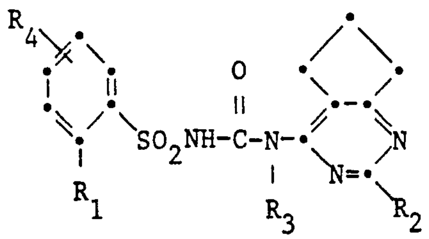

| No. | $R_1$ | $R_2$ | $R_3$ | $R_4$ | phys. Daten Smp. |
|---|---|---|---|---|---|
| 4.001 | $COOCH_3$ | $OCH_3$ | H | H | 179 - 180° Z. |
| 4.002 | $COOCH_3$ | $OCH_2CF_3$ | H | H | 198 - 199° |
| 4.003 | $COOCH_3$ | $OCH_2CH_3$ | H | H | 178 - 180° Z. |
| 4.004 | $COOCH_3$ | $N(CH_3)_2$ | H | H | |
| 4.005 | $COOCH_3$ | $SCH_3$ | H | H | |
| 4.006 | $COOCH_3$ | Cl | H | H | |
| 4.007 | $COOCH_3$ | $CH_3$ | H | H | 177 - 178° Z |
| 4.008 | $NO_2$ | $OCH_3$ | H | H | |
| 4.009 | $NO_2$ | $OCH_2CF_3$ | H | H | |
| 4.010 | $NO_2$ | Cl | H | H | |
| 4.011 | $NO_2$ | $OCH_2CH_3$ | H | H | |
| 4.012 | Cl | $OCH_3$ | H | H | |
| 4.013 | Cl | $OCH_2CF_3$ | H | H | |
| 4.014 | Cl | $OCH_2CH_3$ | H | H | |
| 4.015 | Cl | Cl | H | H | |
| 4.016 | $OCHF_2$ | $OCH_3$ | H | H | |
| 4.017 | $OCHF_2$ | $OCH_2CF_3$ | H | H | |
| 4.018 | $OCHF_2$ | $OCH_2CH_3$ | H | H | |
| 4.019 | $OCHF_2$ | Cl | H | H | |
| 4.020 | $OCHF_2$ | $CH_3$ | H | H | |

**Formulierungsbeispiele**

**Beispiel 2**: Formulierungsbeispiele für Wirkstoffe der Formel I

(% = Gewichtsprozent

| a) Spritzpulver | a) | b) | c) |
|---|---|---|---|
| Wirkstoff | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7-8 Mo AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff | 10 % | 1 % |
| Octylphenolpolyäthylenglykoläther (4-5 Mol AeO) | 3 % | 3 % |
| Ca-Dodecylbenzolsulfonat | 3 % | 3 % |

| | | |
|---|---|---|
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| d) Extruder-Granulat | a) | b) |
|---|---|---|
| Wirkstoff | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungsgranulat | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff | 40 % | 5 % |
| Äthylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37 %-ige wässrige Formaldehyd-Lösung | 0,2 % | 0,2 % |
| Silikonöl in Form einer 75 %-igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| g) Salzlösung | |
|---|---|
| Wirkstoff | 5 % |
| Isopropylamin | 1 % |
| Octylphenolpolyäthylenglykoläther (78 Mol AeO) | 3 % |
| Wasser | 91 % |

**Beispiel 3**: Biologische Versuche

Nachweis der Herbizidwirkung vor dem auflaufen der Pflanzen

Kunststofftöpfe werden mit expandiertem Vermiculit (Dichte: 0,135 g/$cm^3$, Wasserabsorptionsvermögen: 0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70 % gehalten. Während der Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Giesswasser 0,5 % eines handelsüblichen Flüssigdüngers (® Greenzit) zugesetzt. 12 Tage nach der Aussaat wird der Versuch ausgewertet und die Wirkung auf die Versuchspflanzen nach dem folgenden Maßstab bewertet:

1: Pflanze nicht gekeimt oder total abgestorben
2-3: sehr starke Wirkung
4-6: mittlere Wirkung
7-8: schwache Wirkung
9: keine Wirkung (wie unbehandelte Kontrolle).

pre-emergente Wirkung:

Konzentration der Wirkstoffemulsion: 70,8 ppm

| Testpflanze / Wirkstoff Nr. | Nasturtium officinalis | Stellaria media | Agrostis tenuis | Digitaria sanguinalis |
|---|---|---|---|---|
| 1.002 | 2 | 2 | 2 | 3 |
| 1.012 | 1 | 3 | 1 | 4 |
| 1.017 | 2 | 2 | 3 | 3 |
| 1.018 | 1 | 1 | 3 | 8 |
| 1.020 | 2 | 2 | 2 | 4 |
| 1.031 | 3 | 5 | 3 | 6 |
| 1.034 | 1 | 2 | 1 | 2 |
| 1.049 | 2 | 2 | 2 | 3 |
| 1.052 | 2 | 1 | 1 | 2 |
| 1.110 | 4 | 4 | 7 | 8 |
| 1.122 | 6 | 7 | 7 | 9 |
| 1.125 | 1 | 2 | 1 | 2 |
| 1.134 | 2 | 8 | 2 | 9 |
| 1.135 | 3 | 3 | 6 | 7 |
| 1.137 | 2 | 2 | 1 | 2 |
| 1.148 | 2 | 3 | 2 | 3 |
| 1.151 | 2 | 2 | 2 | 3 |
| 1.163 | 2 | 2 | 3 | 3 |
| 1.168 | 2 | 2 | 2 | 6 |
| 1.183 | 1 | 1 | 1 | 2 |
| 1.198 | 1 | 2 | 1 | 3 |
| 1.202 | 2 | 6 | 2 | 7 |
| 1.205 | 1 | 2 | 2 | 3 |
| 1.206 | 1 | 2 | 2 | 3 |
| 1.237 | 2 | 2 | 2 | 2 |
| 1.238 | 1 | 1 | 2 | 3 |
| 1.246 | 2 | 3 | 2 | 6 |
| 1.247 | 2 | 2 | 4 | 4 |
| 1.248 | 2 | 3 | 2 | 4 |
| 1.249 | 2 | 3 | 4 | 5 |
| 1.251 | 2 | 3 | 4 | 4 |
| 1.252 | 2 | 2 | 2 | 3 |
| 1.253 | 4 | 7 | 5 | 8 |
| 1.255 | 3 | 5 | 4 | 6 |
| 1.257 | 2 | 2 | 3 | 3 |
| 4.002 | 2 | 2 | 2 | 3 |
| 4.003 | 3 | 8 | 3 | 9 |

Nachweis der Herbizidwirkung vor dem Auflaufen der Pflanzen

Im Gewächshaus werden Pflanzensamen in Blumentöpfe von 12 - 15 cm Durchmesser gesät. Unmittelbar danach wird die Erdoberfläche mit einer wässrigen Dispersion oder Lösung der Wirkstoffe, erhalten aus einem flowable oder Spritzpulver, behandelt. Es werden verschiedene Konzentrationen mit Wirkstoffmengen pro Hektar angewendet. Die Töpfe werden dann im Gewächshaus bei einer Temperatur von 22 - 25° und 50 - 70 % relativer Luftfeuchtigkeit gehalten. Nach 3 Wochen zeigten die geprüften Verbinungen der Tabellen 1 - 4 gute Wirkung bei Konzentrationen von 0,03 bis 1 kg pro Hektar.

Nachweis der Herbizidwirkung nach dem Auflaufen der Pflanzen (Kontaktwirkung)

Eine grössere Anzahl Unkräuter und Kulturpflanzen, sowohl monokotyle wie dikotyle, werden nach dem Auflaufen, im 4- bis 6-Blattstadium mit einer wässrigen Wirkstoffdispersion in verschiedenen Dosierungen auf die Pflanzen gespritzt und diese bei 24 bis 26° und 45 - 60 % relativer Luftfeuchtigkeit gehalten. 15 Tage nach der Behandlung zeigen die geprüften Verbindungen der Tabellen 1 - 4 gute Herbizidwirkung.

Nachweis der Wuchshemmung bei tropischen Bodenbedecker-Leguminosen (cover crops)

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21° bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Tabellen 1 - 4 behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt werden.

Nachweis der Wuchshemmung bei Getreide

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60 - 90 % der Kontrolle), sowie teilweise eine Zunahme der Stengeldurchmesser auf.

Nachweis der Wuchshemmung bei Gräsern

In Kunststoffschalen mit Erde-Torf-Sand-Gemisch (6 : 3 : 1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt.

Die geprüften Verbindungen der Tabellen 1 - 4 bewirken eine Reduktion des Neuzuwachses im Vergleich zur unbehandelten Kontrolle.

**Patentansprüche**

1. N-Pyrimidin-4-yl-N'-sulfonylharnstoffe der Formel

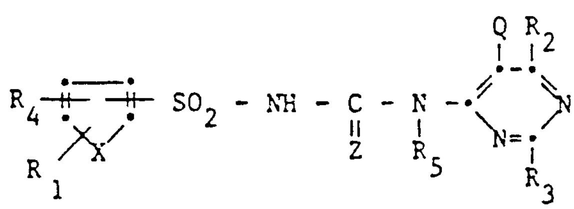

(I),

worin

Q Halogen, $C_1$-$C_4$ Alkyl, $C_1$-$C_4$ Halogenalkyl, $C_1$-$C_4$ Alkoxy, $C_1$-$C_4$-Halogenalkoxy, Amino, $C_1$-$C_4$ Alkylamino, $C_1$-$C_4$ Dialkylamino, $C_4$ Alkoxycarbonyl, Formyl, $C_2$-$C_4$ Alkoxyalkyl, $C_1$-$C_4$-Alkylthio, $C_1$-$C_4$ Alkylsulfinyl, $C_4$ Alkylsulfonyl, $C_1$-$C_4$-Alkenyl oder $C_1$-$C_4$-Alkinyl

$R_1$ Wasserstoff, Halogen, Nitro oder einen Rest $-\underset{R_6}{C}(OC_1-C_4\ Alkyl)_2$,

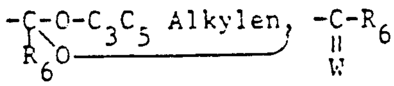, $-SO_2NR_7R_8$ oder $-COR_9$ oder $-(Y)_m-R_{10}$,

$R_2$ und $R_3$ unabhängig voneinander Wasserstoff, Halogen, $C_1$-$C_4$ Alkyl, $C_1$-$C_4$ Alkoxy, $C_1$-$C_4$ Halogenalkyl, $C_1$-$C_4$ Halogenalkoxy, $C_1$-$C_4$ Alkylthio, $C_2$-$C_4$ Alkoxyalkyl, $C_2$-$C_4$ Alkoxyalkoxy, Amino, $C_1$-$C_4$ Alkylamino, $C_1$-$C_4$ Dialkylamino oder Cyclopropyl, wobei Q und $R_2$ zusammen auch eine 2 - 4 gliedrige Kohlenstoff-Kette bilden können, welche gegebenenfalls noch ein Sauerstoff- oder Schwefelatom oder die Gruppe N-$R_5$ enthalten kann,

$R_4$ Wasserstoff, Halogen, $C_1$-$C_4$ Alkyl, Methoxy, Nitro oder Trifluormethyl,

$R_5$ Wasserstoff oder $C_1$-$C_4$ Alkyl oder $C_3$-$C_4$ Alkenyl,

X Sauerstoff, Schwefel, -$NR_5$-, 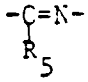, -CH=CH- oder einen annelierten Phenylring,

Z Sauerstoff oder Schwefel,

$R_6$ Wasserstoff, $C_1$-$C_4$ Alkyl, $C_1$-$C_4$-Halogenalkyl, $C_3$-$C_6$ Cycloalkyl, $C_4$-$C_7$ Cycloalkylalkyl oder $C_2$-$C_4$ Alkoxyalkyl,

$R_7$ Wasserstoff, $C_1$-$C_4$ Alkyl, $C_1$-$C_4$-Cyanoalkyl, Methoxy oder Äthoxy,

$R_8$ dasselbe wie $R_5$ oder

$R_7$ und $R_8$ zusammen mit dem sie bindenden Stickstoffatom einen 5 - 6 gliedrigen gesättigten, gegebenenfalls ein Sauerstoff- oder Schwefelatom als Ringglied enthaltenden, Heterocyclus,

$R_9$ $C_1$-$C_4$ Alkoxy, $C_3$-$C_6$ Alkenyloxy, $C_3$-$C_6$ Alkinyloxy, $C_2$-$C_6$-Halogenalkoxy, $C_1$-$C_4$ Cyanoalkoxy, $C_1$-$C_4$ Alkylthio, $C_3$-$C_4$ Alkenylthio, $C_3$-$C_4$ Alkinylthio, $C_5$-$C_6$ Cycloalkoxy, $C_4$-$C_7$ Cycloalkylalkoxy, -$NR_7R_8$ oder $C_2$-$C_6$ Alkoxyalkoxy,

$R_{10}$ -$C_1$-$C_4$ Alkyl, unsubstituiert oder ein- oder mehrfach durch Halogen, Cyano, Methoxy, Äthoxy, Nitro, $C_1$-$C_2$Alkyl $S(O)_n$, $C_1$-$C_2$ Halogenalkoxy, $C_1$-$C_2$ Halogenalkylthio, -$COR_6$, -$COR_9$, -$SO_2NR_7R_8$ -$C_2$-$C_4$ Alkenyl unsubstituiert oder ein- oder mehrfach substituiert durch Halogen, Nitro, Cyan, Methoxy, Äthoxy oder $C_1$-$C_2$, Alkyl$S(O)_n$, -$C_2$-$C_4$ Alkinyl,

m 0 oder 1,

n 0, 1 oder 2

W Sauerstoff oder die Gruppe =N-O-$R_5$ und

Y Sauerstoff, Schwefel -SO- oder -$SO_2$-

bedeuten, mit der Massgabe, dass im Rest $R_1$ in der Gruppe -$(Y)_m$- $R_{10}$,$R_{10}$ bloss $C_3$-$C_4$ Alkinyl ist, wenn m die Zahl 1 bedeutet.

2. N-Pyrimidin-4-yl-N'-sulfonylharnstoffe gemäss Anspruch 1 der Formel Ia

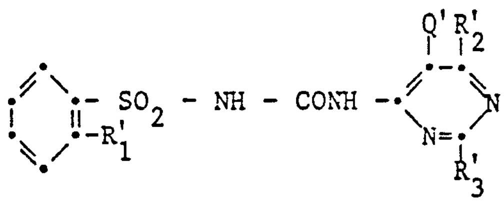 (Ia) ,

worin Q', $R_1$' und $R_2$' die im Anspruch 1 für Q, $R_1$ und $R_2$ gegebene Bedeutung haben, während $R_3$' $C_1$-$C_2$ Alkyl, $C_1$-$C_2$ Halogenalkyl, Cyclopropyl $C_1$-$C_3$ Alkoxy, $C_1$-$C_2$ Halogenalkoxy, Methylthio oder Difluormethylthio bedeutet.

3. N-Pyrimidin-4-yl-N'-sulfonylharnstoffe der Formel Ia

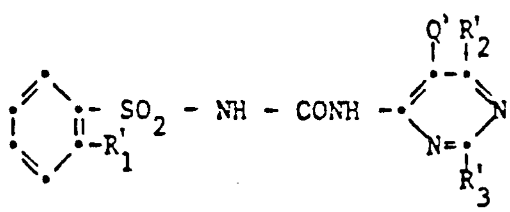 (Ia) ,

worin

Q Halogen, $C_1$-$C_4$-Alkyl, Amino, Formyl, Methylthio oder Methylsulfinyl,

R'$_1$ Halogen, Nitro, $C_1$-$C_4$-Halogenalkyl, $C_1$-$C_4$-Alkoxy, $C_1$-$C_4$-Halogenalkoxy, $C_3$-$C_4$-Alkenyloxy, $C_2$-$C_4$-Halogenalkenyloxy, $C_1$-$C_4$-Halogenalkylthio, $C_1$-$C_4$-Alkoxycarbonyl, $C_1$-$C_4$-Alkylsulfonyl oder Dimethylsulfamoyl,

R'$_2$ Halogen, $C_1$-$C_3$-Alkyl, Cyclopropyl, $C_1$-$C_3$-Alkoxy, $C_1$-$C_2$-Halogen-alkoxy, amino, Methylamino oder Dimethylamino,

R'$_3$ $C_1$-$C_2$-Alkyl, $C_1$-$C_2$ Halogenalkyl, Cyclopropyl, $C_1$-$C_3$-Alkoxy, $C_1$-$C_2$-Halogenalkoxy, Methylthio oder Difluormethylthio bedeuten.

4. N-Pyrimidin-4-yl-N'-sulfonylharnstoffe gemäss Anspruch 3 der Formel Ia

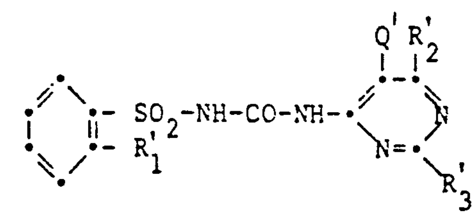

(Ia) ,

worin

Q' Halogen, $C_1$-$C_4$ Alkyl, Amino, Formyl, Methylthio oder Methylsulfinyl,

$R_1$' Halogen, Nitro, $C_1$-$C_4$-Halogenalkyl, $C_1$-$C_4$-Alkoxy, $C_1$-$C_4$-Halogenalkoxy, $C_1$-$C_4$-Halogenalkylthio, $C_1$-$C_4$-Alkoxycarbonyl oder Dimethylsulfamoyl,

$R_2$' Halogen, $C_1$-$C_2$-Alkyl, $C_1$-$C_2$-Alkoxy, $C_1$-$C_2$-Halogenalkoxy, Methylamino oder Dimethylamino,

$R_3$' $C_1$-$C_2$-Alkoxy, Trifluoräthoxy oder Difluormethoxy, bedeuten.

5. N-(2-Methoxycarbonylphenylsulfonyl)-N'-(2,6-dimethoxy-5-methylpyrimidin-4-yl)-harnstoff gemäss Anspruch 1.

6. N-(2-Difluormethoxyphenylsulfonyl)-N'-(2,6-dimethoxy-5-methylpyrimidin-4-yl)-harnstoff gemäss Anspruch 3.

7. Verfahren zur Herstellung der N-Pyrimidin-4-yl-N'-sulfonylharnstoffe der Formel I oder Ia, dadurch gekennzeichnet, dass man ein Sulfonamid der Formel II

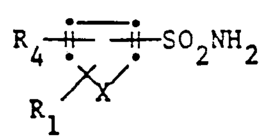

(II) ,

worin $R_1$ und $R_4$ die im Anspruch 1 oder 3 gegebene Bedeutung haben, in Gegenwart einer Base mit einem Pyrimidin-4-yl-carbamat der Formel III umsetzt

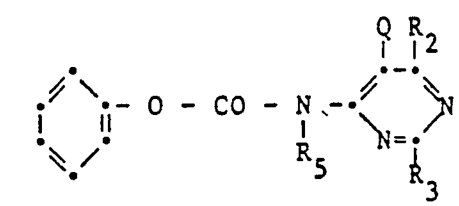

(III) ,

worin Q, $R_2$, $R_3$ und $R_5$ die unter Formel II gegebene Bedeutung haben, und gegebenenfalls in ein Salz überführt.

8. Verfahren zur Herstellung der Verbindungen der Formel I oder Ia gemäss Anspruch 1 oder 3, dadurch gekennzeichnet, dass man ein Phenylsulfonylisocyanat der Formel IV

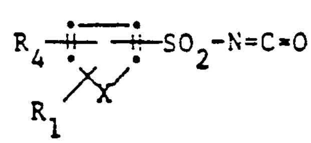

(IV),

worin $R_1$, $R_4$ und X die unter Formel I oder Ia gegebene Bedeutung haben, gegebenenfalls in Gegenwart einer Base mit einem Amin der Formel V umsetzt

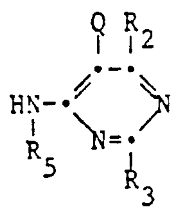

(V),

worin Q, $R_2$, $R_3$ und $R_5$ die im Anspruch 1 oder 3 gegebene Bedeutung haben und gegebenenfalls in ein Salz überführt.

9. Verfahren zur Herstellung der Verbindungen der Formel I oder Ia gemäss Anspruch 1 oder 3, worin $R_5$ Wasserstoff bedeutet, dadurch gekennzeichnet, dass man ein Sulfonamid der Formel II, gemäss Anspruch 7 gegebenenfalls in Gegenwart einer Base mit einem Isocyanat der Formel VI umsetzt,

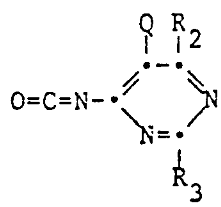

(VI),

worin Q, $R_2$ und $R_3$ die unter Formel I oder Ia gegebene Bedeutung haben, umsetzt und gegebenenfalls in ein Salz überführt.

10 Verfahren zur Herstellung der Verbindungen der Formel I oder Ia gemäss Anspruch 1 oder 3, dadurch gekennzeichnet, dass man ein N-Phenylsulfonylcarbamat der Formel VII

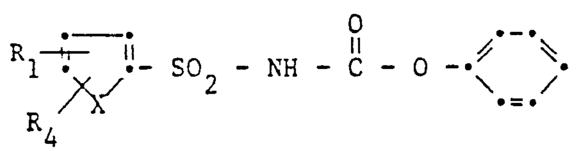

(VII),

worin $R_1$, $R_4$ und X die unter Formel I gegebene Bedeutung haben, mit einem Amin der Formel V gemäss Anspruch 8, umsetzt und gegebenfalls in ein Salz überführt.

11. Verfahren zur Herstellung von basischen Additionssalzen der Formel I oder Ia gemäss einem der Ansprüche 7 bis 10, dadurch gekennzeichnet, dass man einen Sulfonylharnstoff der Formel I oder Ia mit einem Amin, einem Alkalimetall- oder Erdalkalimetallhydroxid oder einer quaternären Ammoniumbase umsetzt.

12. Ein herbizides und den Pflanzenwuchs regulierendes bzw. hemmendes Mittel, dadurch gekennzeichnet, dass es neben Träger- und/oder anderen Zuschlagstoffen als Wirkstoff einen N-Pyrimidin-4-yl-N'-sulfonylharnstoff der Formel I oder Ia gemäss Anspruch 1 oder 3 enthält.

13. Die Verwendung der N-Pyrimidin-4-yl-N'-sulfonyl-harnstoffe der Formel I oder Ia, Anspruch 1 oder 3, oder sie enthaltende Mittel zur Bekämpfung unerwünschten Pflanzenwachstums.

14. Die Verwendung der N-Pyrimidin-4-yl-N'-sulfonyl-harnstoffe der Formel I oder Ia, Anspruch 1 oder 3, oder sie enthaltender Mittel zur Hemmung des Pflanzenwachstums.

15. Die Verwendung der N-Pyrimidin-4-yl-N'-sulfonyl-harnstoffe der Formel I oder Ia, oder sie enthaltender Mittel gemäss Anspruch 1 oder 3, zur selektiven pre- oder post-emergenten Bekämpfung von Unkräutern in Nutzpflanzenkulturen.

16. Verfahren zur selektiven Bekämpfung von Unkräutern in Nutzpflanzenkulturen, dadurch gekennzeichnet, dass man die Kulturen oder deren Anbaufläche mit einer wirksamen Menge eines N-Pyrimidin-4-yl-N'-sulfonylharnstoffs der Formel I oder Ia, Anspruch 1 oder 3, behandelt.

## Claims

1. N-pyrimidin-4-yl-N,-sulfonylureas of formula

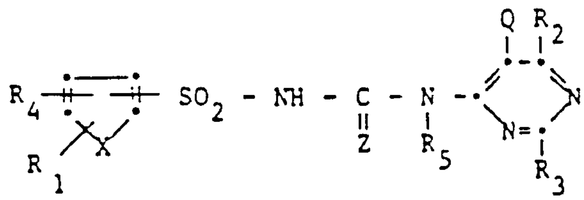

(I)

wherein

Q is halogen, $C_1$-$C_4$alkyl, $C_1$-$C_4$haloalkyl, $C_1$-$C_4$alkoxy, $C_1$-$C_4$haloalkoxy, amino, $C_1$-$C_4$alkylamino, $C_1$-$C_4$dialkylamino, $C_4$alkoxycarbonyl, formyl, $C_2$-$C_4$alkoxyalkyl, $C_1$-$C_4$alkylthio, $C_1$-$C_4$alkylsulfinyl, $C_4$alkylsulfonyl, $C_1$-$C_4$alkenyl or $C_1$-$C_4$alkynyl,

$R_1$ is hydrogen, halogen, nitro or a radical

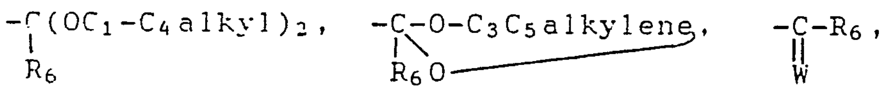

$-SO_2NR_7R_8$, $-COR_9$- or $-(Y)_m-R_{10}$,

$R_2$ and $R_3$ independently of one another are each hydrogen, halogen, $C_1$-$C_4$alkyl, $C_1$-$C_4$alkoxy, $C_1$-$C_4$haloalkyl, $C_1$-$C_4$haloalkoxy, $C_1$-$C_4$alkylthio, $C_2$-$C_4$alkoxyalkyl, $C_2$-$C_4$alkoxyalkoxy, amino, $C_1$-$C_4$ alkylamino, $C_1$-$C_4$dialkylamino or cyclopropyl, and Q and $R_2$ together may also form a 2- to 4-membered carbon chain, which may optionally also contain an oxygen or sulfur atom or the group N-$R_5$,

$R_4$ is hydrogen, halogen, $C_1$-$C_4$alkyl, methoxy, nitro or trifluoromethyl,

$R_5$ is hydrogen, $C_1$-$C_4$alkyl or $C_3$-$C_4$alkenyl,

X is oxygen, sulfur, $-NR_5$-, 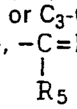, -CH=CH- or a fused phenyl ring,

Z is oxygen or sulfur,

$R_6$ is hydrogen, $C_1$-$C_4$alkyl, $C_1$-$C_4$haloalkyl, $C_3$-$C_6$cycloalkyl, $C_4$-$C_7$cycloalkylalkyl or $C_2$-$C_4$alkoxyalkyl,

$R_7$ is hydrogen, $C_1$-$C_4$alkyl, $C_1$-$C_4$cyanoalkyl, methoxy or ethoxy,

$R_8$ is the same as $R_5$, or

$R_7$ and $R_8$ together with the nitrogen atom linking them form a 5- or 6-membered saturated heterocycle optionally containing an oxygen or sulfur atom as ring member,

$R_9$ is $C_1$-$C_4$alkoxy, $C_3$-$C_6$alkenyloxy, $C_3$-$C_6$alkynyloxy, $C_2$-$C_6$haloalkoxy, $C_1$-$C_4$cyanooalkoxy, $C_1$-$C_4$alkylthio, $C_3$-$C_4$alkenylthio, $C_3$-$C_4$alkynylthio, $C_5$-$C_6$cycloalkoxy, $C_4$-$C_7$cycloalkylalkoxy, $-NR_7R_8$ or $C_2$-$C_6$alkoxyalkoxy,

$R_{10}$ is $C_1$-$C_4$alkyl, unsubstituted or mono- or poly-substituted by halogen, cyano, methoxy, ethoxy, nitro, $C_1$-$C_2$alkyl-$S(O)_n$, $C_1$-$C_2$haloalkoxy-, $C_1$-$C_2$haloalkylthio, $-COR_6$, $-COR_9$ or $-SO_2NR_7R_8$, or is $C_2$-$C_4$alkenyl, unsubstituted or mono- or poly-substituted by halogen, nitro, cyano, methoxy, ethoxy or $C_1$-$C_2$alkyl-$S(O)_n$, or is $C_2$-$C_4$alkynyl,

m is 0 or 1,

n is 0, 1 or 2,

W is oxygen or the group =N-O-$R_5$, and

Y is oxygen, sulfur, -SO- or $-SO_2$-,

with the proviso that in the radical $R_1$ in the group $-(Y)_m-R_{10}$, $R_{10}$ is only $C_3$-$C_4$alkynyl when m is 1.

2. N-pyrimidin-4-yl-N'-sulfonylureas according to claim 1 of formula Ia

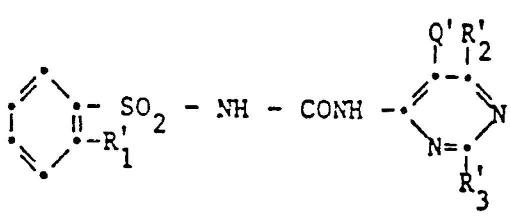

(Ia)

wherein Q', $R_1$' and $R_2$' have the meanings given in claim 1 for Q, $R_1$ and $R_2$, whilst $R_3$' is $C_1$-$C_2$alkyl, $C_1$-$C_2$haloalkyl, cyclopropyl, $C_1$-$C_3$alkoxy, $C_1$-$C_2$haloalkoxy, methylthio or difluoromethylthio.

3. N-pyrimidin-4-yl-N'-sulfonylureas of formula Ia

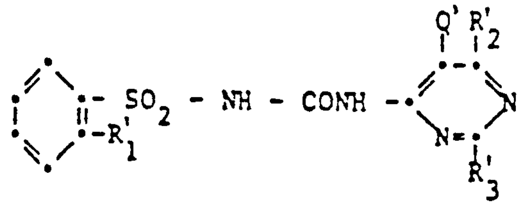

(Ia)

wherein

Q' is halogen, $C_1$-$C_4$alkyl, amino, formyl, methylthio or methylsulfinyl,

$R_1$' is halogen, nitro, $C_1$-$C_4$haloalkyl, $C_1$-$C_4$alkoxy, $C_1$-$C_4$haloalkoxy, $C_3$-$C_4$alkenyloxy, $C_2$-$C_4$haloalkenyloxy-, $C_1$-$C_4$haloalkylthio, $C_1$-$C_4$alkoxycarbonyl, $C_1$-$C_4$alkylsulfonyl or dimethylsulfamoyl,

$R_2$' is halogen, $C_1$-$C_3$alkyl, cyclopropyl, $C_1$-$C_3$alkoxy, $C_1$-$C_2$haloalkoxy, amino, methylamino or dimethylamino,

$R_3$' is $C_1$-$C_2$alkyl, $C_1$-$C_2$haloalkyl, cyclopropyl, $C_1$-$C_3$-alkoxy, $C_1$-$C_2$haloalkoxy, methylthio or difluoromethylthio.

4. N-pyrimidin-4-yl-N'-sulfonylureas according to claim 3 of formula Ia

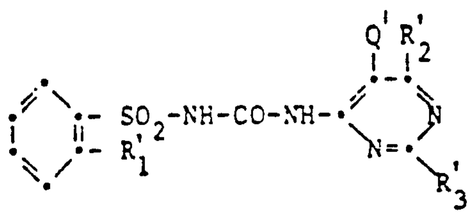

(Ia)

wherein

Q' is halogen, $C_1$-$C_4$alkyl, amino, formyl, methylthio or methylsulfinyl,

$R_1$' is halogen, nitro, $C_1$-$C_4$haloalkyl, $C_1$-$C_4$alkoxy, $C_1$-$C_4$-haloalkoxy, $C_1$-$C_4$haloalkylthio, $C_1$-$C_4$alkoxycarbonyl or dimethylsulfamoyl,

$R_2$' is halogen, $C_1$-$C_2$alkyl, $C_1$-$C_2$alkoxy, $C_1$-$C_2$haloalkoxy, methylamino or dimethylamino,

$R_3$' is $C_1$-$C_2$alkoxy, trifluoroethoxy or difluoromethoxy.

5. N-(2-methoxycarbonylphenylsulfonyl)-N'-(2,6-dimethoxy-5-methylpyrimidin-4-yl)-urea according to claim 1.

6. N-(2-difluoromethoxyphenylsulfonyl)-N'-(2,6-dimethoxy-5-methylpyrimidin-4-yl)-urea according to claim 3.

7. A process for the preparation of N-pyrimidin-4-yl-N'-sulfonylureas of formula I or Ia, which process comprises reacting a sulfonamide of formula II

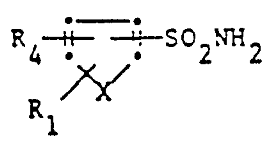

(II)

wherein $R_1$ and $R_4$ are as defined in claim 1 or 3, in the presence of a base, with a pyrimidin-4-yl-carbamate of formula III

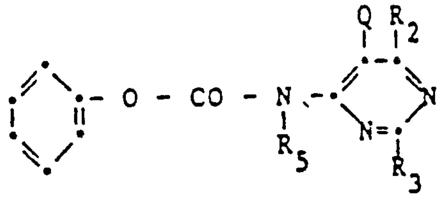

(III),

wherein Q, $R_2$, $R_3$ and $R_5$ are as defined for formula I or Ia, and optionally converting the product obtained into a salt.

8. A process for the preparation of compounds of formula I or Ia according to claim 1 or 3, which process

comprises reacting a phenylsulfonylisocyanate of formula IV

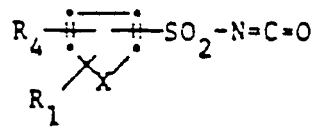

(IV),

wherein $R_1$, $R_4$ and X are as defined for formula I or Ia, optionally in the presence of a base, with an amine of formula V

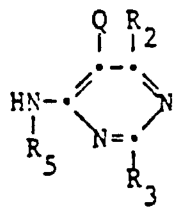

(V)

wherein Q, $R_2$, $R_3$ and $R_5$ are as defined in claim 1 or 3, and optionally converting the product obtained into a salt.

9. A process for the preparation of compounds of formula I or Ia according to claim 1 or 3, in which $R_5$ is hydrogen, which process comprises reacting a sulfonamide of formula II according to claim 7, optionally in the presence of a base, with an isocyanate of formula VI

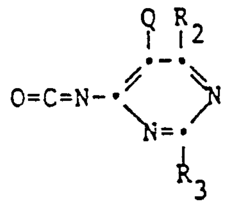

(VI),

wherein Q, $R_2$ and $R_3$ are as defined for formula I or Ia, and optionally converting the product obtained into a salt.

10. A process for the preparation of compounds of formula I or Ia according to claim 1 or 3, which process comprises reacting an N-phenylsulfonylcarbamate of formula VII

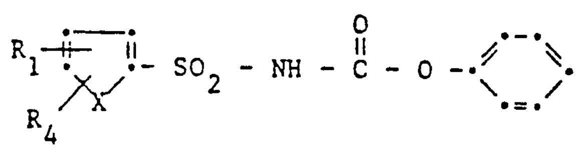

(VII),

wherein $R_1$, $R_4$ and X are as defined for formula I, with an amine of formula V according to claim 8, and optionally converting the product obtained into a salt.

11. A process for the preparation of basic addition salts of formula I or Ia according to any one of claims 7 to 10, which process comprises reacting a sulfonylurea of formula I or Ia with an amine, with an alkali metal hydroxide or alkaline earth metal hydroxide or with a quaternary ammonium base.

12. A herbicidal and plant growth-regulating or -inhibiting composition which contains, as active ingredient, an N-pyrimidin-4-yl-N'-sulfonylurea of formula I or Ia according to claim 1 or 3, together with carriers and/or other additives.

13. The use of the N-pyrimidin-4-yl-N'-sulfonylureas of formula I or Ia, claim 1 or 3, or of compositions containing them, for controlling undesired plant growth.

14. The use of the N-pyrimidin-4-yl-N'-sulfonylureas of formula I or Ia, claim 1 or 3, or of compositions containing them, for inhibiting plant growth.

15. The use of the N-pyrimidin-4-yl-N'-sulfonylureas of formula I or Ia or of compositions containing them according to claim 1 or 3 for the selective control, pre- or post-emergence, of weeds in crops of useful plants.

16. A method of selectively controlling weeds in crops of useful plants, which method comprises treating the crops or the cultivated area thereof with an effective amount of an N-pyrimidin-4-yl-N'-sulfonylurea of formula I or Ia, claim 1 or 3.

## Revendications

1. - N-pyrimidine-4-yl-N'-sulfonyrlurées de formule

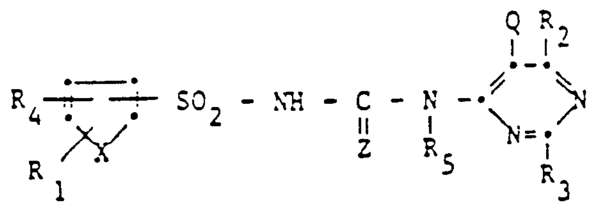 (I),

dans laquelle

Q représente un halogène, un alkyle en $C_1$-$C_4$, un halogénoalkyle en $C_1$-$C_4$, un alcoxy en $C_1$-$C_4$, un halogénoalcoxy en $C_1$-$C_4$, un amino, un alkylamino en $C_1$-$C_4$, un dialkylamino en $C_1$-$C_4$, un alcoxycarbonyle en $C_4$, le formyle, un alcoxyalkyle en $C_2$-$C_4$, un alkylthio en $C_1$-$C_4$, un alkylsulfinyle en $C_1$-$C_4$, un alkylsulfonyle en $C_4$, un alcényle en $C_1$-$C_4$ ou un alcynyle $C_1$-$C_4$,

$R_1$ l'hydrogène, un halogène, un nitro ou un reste

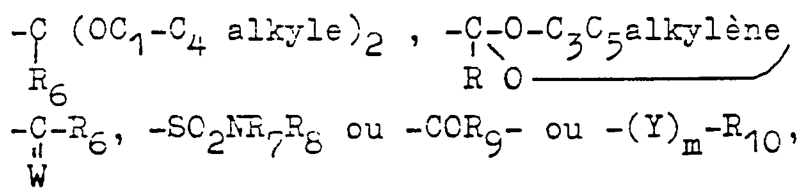

$R_2$ et $R_3$ représentent indépendamment l'un de l'autre, l'hydrogène, un halogène, un alkyle en $C_1$-$C_4$, un alcoxy en $C_1$-$C_4$, un halogénoalkyle en $C_1$-$C_4$, un halogénoalcoxy en $C_1$-$C_4$, un alkylthio en $C_1$-$C_4$, un alcoxyalkyle en $C_2$-$C_4$, un alcoxyalcoxy en $C_2$-$C_4$, un amino, un alkylamino en $C_1$-$C_4$, un dialkylamino en $C_1$-$C_4$ ou un cyclopropyle, Q et $R_2$ pouvant également former ensemble une chaîne carbonée à 2 - 4 chaînons, ladite chaîne pouvant éventuellement contenir encore un atome d'oxygène ou de soufre ou le groupe N-$R_5$,

$R_4$ l'hydrogène, un halogène, un alkyle en $C_1$-$C_4$, un méthoxy, un nitro ou un trifluorométhyle,

$R_5$ l'hydrogène ou un alkyle en $C_1$-$C_4$ ou un alcényle en $C_3$-$C_4$,

X l'oxygène, le soufre, -$NR_5$-,

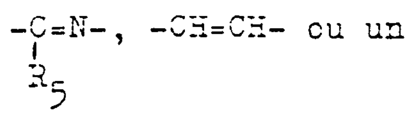

noyau phényle condensé,

Z l'oxygène ou le soufre,

$R_6$ l'hydrogène, un alkyle en $C_1$-$C_4$, un halogénoalkyle $C_1$-$C_4$, un cycloalkyle en $C_3$-$C_6$, un cycloalkylalkyle en $C_4$-$C_7$ ou un alcoxyalkyle en $C_2$-$C_4$,

$R_7$ l'hydrogène, un alkyle en $C_1$-$C_4$, un cyanoalkyle en $C_1$-$C_4$, un méthoxy ou un éthoxy,

$R_8$ la même signification que $R_5$ ou

$R_7$ et $R_8$ forment ensemble avec l'atome d'azote les reliant un hétérocycle à 5 - 6 chaînons saturés, contenant éventuellement un atome d'oxygène ou de soufre comme terme cyclique,

$R_9$ un alcoxy en $C_1$-$C_4$, un alcényloxy en $C_3$-$C_6$ un alcynyloxy en $C_3$-$C_6$, un halogénoalcoxy en $C_2$-$C_6$, un cyanoalcoxy en $C_1$-$C_4$, un alkylthio en $C_1$-$C_4$, un alcénylthio en $C_3$-$C_4$, un alcynylthio en $C_3$-$C_4$, un cycloalcoxy en $C_5$-$C_6$, un cycloalkylalcoxy en $C_4$-$C_7$, -$NR_7R_8$ ou un alcoxyalcoxy en $C_2$-$C_6$,

$R_{10}$ un alkyle en $C_1$-$C_4$, non substitué ou une ou plusieurs fois substitué par un halogène, un cyano, un méthoxy, un éthoxy, un nitro, $C_1$-$C_2$alkyl $S(O)_n$, un halogénoalcoxy en $C_1$-$C_2$, un halogénoalkylthio en $C_1$-$C_2$, -$COR_6$, -$COR_9$, -$SO_2NR_7R_8$, un alcényle en $C_2$-$C_4$ non substitué ou une ou plusieurs fois substitué par un halogène, un nitro, un cyano, un méthoxy, un éthoxy ou $C_1$-$C_2$ alkyl $S(O)_n$, un alcynyle en -$C_2$-$C_4$,

m est 0 ou 1,

n 0, 1 ou 2

W l'oxygène, le soufre, -SO- ou -$SO_2$-

sous réserve que dans le reste $R_1$ dans le groupe -$(Y)_m$-$R_{10}$, $R_{10}$ est simplement un alcynyle en $C_3$-$C_4$, lorsque

m est égal au chiffre 1.

2. N-pyrimidine-4-yl-N'-sulfonylurées selon la revendication 1 de formule Ia

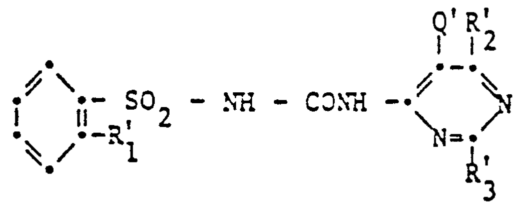

(Ia) ,

où Q', R'$_1$ et R'$_2$ ont la signification donnée dans la revendication 1 pour Q, $R_1$ et $R_2$, tandis que R'$_3$ représente un alkyle en $C_1$-$C_2$, un halogénoalkyle en $C_1$-$C_2$, le cyclopropyle, un alcoxy en $C_1$-$C_3$, un halogénoalcoxy en $C_1$-$C_2$, le méthylthio ou le difluorométhylthio.

3. N-pyrimidine-4-yl-N'-sulfonylurées de formule Ia

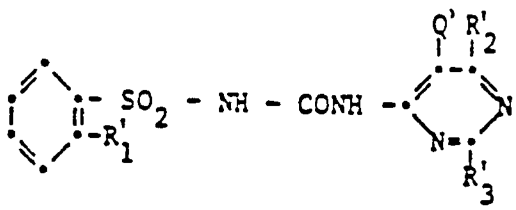

(Ia) ,

où

R' représente un halogène, un alkyle en $C_1$-$C_4$, un amino, le formyle, le méthylthio ou le méthylsulfinyle,

R'$_1$ un halogène, un nitro, un halogénoalkyle en $C_1$-$C_4$, un alcoxy en $C_1$-$C_4$, un halogénoalcoxy en $C_1$-$C_4$, un alcényloxy en $C_3$-$C_4$, un halogénoalcényloxy en $C_2$-$C_4$, un halogénoalkylthio en $C_1$-$C_4$, un alcoxycarbonyle en $C_1$-$C_4$, un alkylsulfonyle en $C_1$-$C_4$ ou le diméthylsulfamoyl,

R'$_2$ un halogène, un alkyle en $C_1$-$C_3$, le cyclopropyle, un alcoxy en $C_1$-$C_3$, un halogénoalcoxy en $C_1$-$C_2$, un amino, le méthylamino ou le diméthylamino,

R'$_3$ un alkyle en $C_1$-$C_2$, un halo génoalkyle en $C_1$-$C_2$, le cyclopropyle, un alcoxy en $C_1$-$C_3$, un halogénoalcoxy en $C_1$-$C_2$, le méthylthio ou le difluorométhylthio.

4. N-pyrimidine-4-yl-N'-sulfonylurées selon la revendication 3 de formule Ia

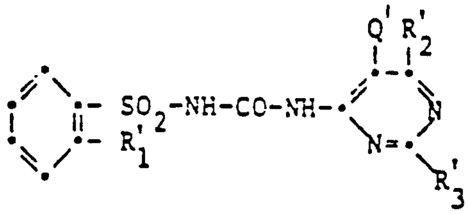

(Ia) ,

où

Q' représente un halogène, un alkyle en $C_1$-$C_4$, un amino, le formyle, le méthylthio ou le méthylsulfinyle,

R'$_1$ un halogène, un nitro, un halogénoalkyle en $C_1$-$C_4$, un alcoxy en $C_1$-$C_4$, un halogénoalcoxy en $C_1$-$C_4$, un halogénoalkylthio en $C_1$ -$C_4$, un alcoxycarbonyle en $C_1$-$C_4$ ou le diméthylsulfamoyl,

R'$_2$ un halogène, un alkyle en $C_1$-$C_2$, un alcoxy en $C_1$-$C_2$, un halogénoalcoxy en $C_1$-$C_2$, le méthylamino ou le diméthylamino,

R'$_3$ un alcoxy en $C_1$-$C_2$, le trifluoroéthoxy ou le difluorométhoxy.

5. N-(2-méthoxycarbonylphénylsulfamoyl)-N'-(2,6-diméthoxy-5-méthyl-pyrimidine-4-yl)-urée selon la revendication 1.

6. N-(2-difluorométhoxyphénylsulfonyl)-N'-(2,6-diméthoxy-5-méthyl-pyrimidine-4-yl)-urée selon la revendication 3.

7. Procédé de préparation des N-pyrimidine-4-yl-N'-sulfonyl-urées de formule I ou Ia, caractérisé en cu que l'on fait réagir un sulfonamide de formule II

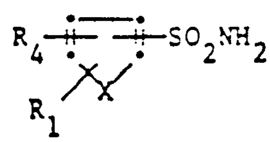

(II),

où $R_1$ et $R_4$ ont la signification donnée dans la revendication 1 ou 3, en présence d'une base, avec un pyrimidine-4-yl-carbamate de formule III

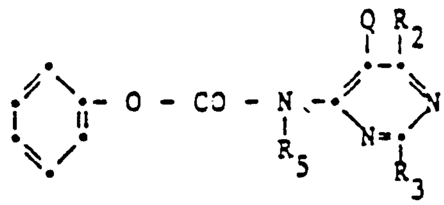

(III),

ou Q, $R_2$, $R_3$ et $R_5$ ont la signification donnée à propos de la formule I ou Ia et éventuellement en ce qu'on le transforme en un sel.

8. Procédé de préparation des composés de formule I ou Ia selon la revendication 1 ou 3, caractérisé en ce que l'on fait réagir un phénylsulfonylisocyanate de formule IV

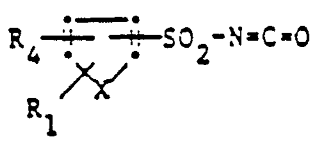

(IV),

où $R_1$, $R_4$ et X ont la signification donnée à propos de la formule I ou Ia, éventuellement en présence d'une base, avec une amine de formule V

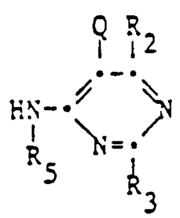

(V),

où Q, $R_2$, $R_3$ et $R_5$ ont la signification donnée dans la revendication 1 ou 3 et éventuellement en ce qu'on le transforme en un sel.

9. Procédé de préparation des composés de formule I ou Ia selon la revendication 1 ou 3 où $R_5$ représente l'hydrogène, caractérisé en ce que l'on fait réagir un sulfonamide de formule II selon la revendication 7, éventuellement en présence d'une base, avec un isocyanate de formule VI

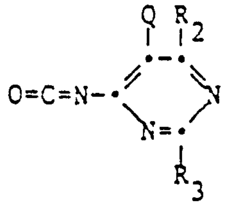

(VI),

où Q, $R_2$ et $R_3$ ont la signification donnée à propos de la formule I ou II, et éventuellement en ce qu'on le transforme en un sel.

10. Procédé de préparation des composés de formule I ou Ia selon la revendication 1 ou 3, caractérisé en ce que l'on fait réagir un N-phénylsulfonylcarbamate de formule VII,

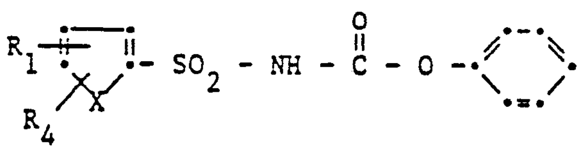

(VII) ,

où $R_1$, $R_4$ et X ont la signification donnée à propos de la formule I, avec une amine de formule V selon la revendication 8 et éventuellement en ce qu'on le transforme en un sel.

11. Procédé de préparation de sels d'addition basiques de formule I ou Ia selon l'une des revendications 7 à 10, caractérisé en ce que l'on fait réagir une sulfonylurée de formule I ou Ia avec une amine, un hydroxyde de métal alcalin ou alcalino-terreux ou une base d'ammonium quaternaire.

12. Agent herbicide et régulateur ou inhibiteur de la croissance des plantes, caractérisé en ce qu'il contient à côté d'un support et/ou d'autres additifs, comme matière active, un N-pyrimidine-4-yl-N'-sulfonylurée de formule I ou Ia selon la revendication 1 ou 3.

13. Utilisation des N-pyrimidine-4-yl-N'-sulfonylurées de formule I ou Ia selon la revendication 1 ou 3 ou agent les contenant pour lutter contre la croissance des plantes indésirées.

14. Utilisation des N-pyrimidine-4-yl-N'-sulfonylurées de formule I ou Ia selon la revendication 1 ou 3 ou un agent les contenant pour inhiber la croissance des plantes.

15. Utilisation des N-pyrimidine-4-yl-N'-sulfonylulrées de formule I ou Ia ou un agent les contenant selon la revendication 1 ou 3, pour la lutte sélective pre- ou post-émergente des mauvaises herbes dans les cultures de plantes utiles.

16. Procédé pour lutter sélectivement contre les mauvaises herbes dans les cultures de plantes utiles, caractérisé en ce que l'on traite les cultures ou les surfaces de ces cultures avec une quantité efficace d'une N-pyrimidine-4-yl-N'-sulfonylurée de formule I ou Ia selon la revendication 1 ou 3.